# EUROPEAN PATENT APPLICATION

(11) **EP 3 385 435 A1**
(43) Date of publication of application: **10.10.2018**
(21) Application number: 17164645.8
(22) Date of filing: 03.04.2017
(51) Int. Cl.: D04H 3/013, D04H 3/016

(54) **NONWOVEN CELLULOSE FIBER FABRIC WITH DIFFERENT SETS OF PORES**

(71) Applicant: LENZING AKTIENGESELLSCHAFT, 4860 Lenzing (AT)
(72) Inventor: Carlyle, Tom, Spanish Fort, AL Alabama 36527 (US); Einzmann, Mirko, 4600 Wels (AT); Goldhalm, Gisela, 3363 Neufurth (AT); Hayhurst, Malcolm John, Bulkington, Warwickshire CV12 9RZ (GB); Mayer, Katharina, 4813 Altmünster (AT); Sagerer Foric, Ibrahim, 4840 Vöcklabruck (AT)
(74) Representative: Dilg, Haeusler, Schindelmann Patentanwaltsgesellschaft mbH

(57) **Abstract**

A nonwoven cellulose fiber fabric (102), in particular directly manufactured from lyocell spinning solution (104), wherein the fabric (102) comprises a network of substantially endless fibers (108), and wherein the fabric (102) further comprises a plurality of first or primary pores (260) delimited between a first plurality of the fibers (108) and having sizes (280) within a first size range, and a plurality of second or secondary pores (264) delimited between a second plurality of the fibers (108) and having sizes (282) within a second size range, wherein the first size range encompasses sizes (280) being smaller than sizes (282) encompassed by the second size range.

## Description

### Field of the invention

The invention relates to a nonwoven cellulose fiber fabric, a method of manufacturing a nonwoven cellulose fiber fabric, a device for manufacturing a nonwoven cellulose fiber fabric, a product or composite, a method of controlling release of an active agent, and a method of use.

### Background of the invention

Lyocell technology relates to the direct dissolution of cellulose wood pulp or other cellulose-based feedstock in a polar solvent (for example n-methyl morpholine n-oxide, which may also be denoted as "amine oxide" or "AO") to produce a viscous highly shear-thinning solution which can be transformed into a range of useful cellulose-based materials. Commercially, the technology is used to produce a family of cellulose staple fibers (commercially available from Lenzing AG, Lenzing, Austria under the trademark TENCEL®) which are widely used in the textile industry. Other cellulose products from lyocell technology have also been used.

Cellulose staple fibers have long been used as a component for conversion to nonwoven webs. However, adaption of lyocell technology to produce nonwoven webs directly would access properties and performance not possible for current cellulose web products. This could be considered as the cellulosic version of the meltblow and spunbond technologies widely used in the synthetic fiber industry, although it is not possible to directly adapt synthetic polymer technology to lyocell due to important technical differences.

Much research has been carried out to develop technology to directly form cellulose webs from lyocell solutions (*inter alia*, WO 98/26122, WO 99/47733, WO 98/07911, US 6,197,230, WO 99/64649, WO 05/106085, EP 1 358 369, EP 2 013 390). Further art is disclosed in WO 07/124521 A1 and WO 07/124522 A1.

### Object and summary of the invention

It is an object of the invention to provide a cellulose-based fabric having adjustable properties in terms of interaction of the fabric with a medium.

In order to achieve the object defined above, a nonwoven cellulose fiber fabric, a method of manufacturing a nonwoven cellulose fiber fabric, a device for manufacturing a nonwoven cellulose fiber fabric, a method of controlling release of an active agent, a product or composite, and a method of use according to the independent claims are provided.

According to an exemplary embodiment of the invention, a (in particular solution-blown) nonwoven cellulose fiber fabric is provided (which is in particular directly (in particular in an *in situ* process or in a continuous process executable in a continuously operating production line) manufactured from lyocell spinning solution), and wherein the fabric further comprises a plurality of first or primary pores delimited between the single fibers and having sizes within a first size range (which may be suitable or configured for retaining and/or releasing first particles (which may optionally form part of the fabric)), and a plurality of second or secondary pores having sizes within a second size range (which may be configured for retaining and/or releasing second particles (which may optionally form part of the fabric)), wherein the first size range encompasses sizes (in particular encompasses only sizes) being smaller than sizes encompassed (in particular sizes only encompassed) by the second size range.

According to another exemplary embodiment, a method of manufacturing (in particular solution-blown) nonwoven cellulose fiber fabric directly from lyocell spinning solution is provided, wherein the method comprises extruding the lyocell spinning solution through a jet with orifices supported by a gas flow into a coagulation fluid atmosphere (in particular an atmosphere of dispersed coagulation fluid) to thereby form substantially endless fibers, collecting the fibers on a fiber support unit to thereby form the fabric, and adjusting process parameters so that the fabric is formed with a plurality of primary pores delimited between a first plurality of the fibers and having sizes within a first size range, and a plurality of secondary pores delimited between a second plurality of the fibers (wherein the secondary pores may be formed for instance after collecting the fibers on the fiber support unit, for example by hydroentanglement) and having sizes within a second size range, wherein the first size range encompasses sizes being smaller than sizes encompassed by the second size range.

According to a further exemplary embodiment, a device for manufacturing (in particular solution-blown) nonwoven cellulose fiber fabric directly from lyocell spinning solution is provided, wherein the device comprises an jet with orifices configured for extruding the lyocell spinning solution supported by a gas flow, a coagulation unit configured for providing a coagulation fluid atmosphere for the extruded lyocell spinning solution to thereby form substantially endless fibers, a fiber support unit configured for collecting the fibers to thereby form the fabric, optionally an aftertreatment device (such as a hydroentanglement and/or needlepunching device), and a control unit (such as a processor configured for executing program code for manufacturing the nonwoven cellulose fiber fabric directly from the lyocell spinning solution) configured for adjusting process parameters so that the fabric is formed with a plurality of primary pores, having sizes within a first size range, and a plurality of secondary pores having sizes within a second size range, wherein the first size range encompasses sizes being smaller than sizes encompassed by the second size range.

According to yet another embodiment, a method of controlling release of an active agent out of a fabric is provided, wherein the method comprises providing a nonwoven cellulose fiber fabric comprising a network of substantially endless fibers, a plurality of pores delimited between the fibers, and the active agent retained in at least part of the pores and/or in (in particular fluidically) connected cavities in the fabric, and adjusting a (for instance physical or chemical) condition of the fabric for triggering release of the active agent out of the pores and/or the cavities (in particular adjusting a humidity state of the fibers for triggering one of the group consisting of a swelling and a shrinkage of the fibers to thereby control release of the active agent out of the pores).

According to yet another embodiment, a nonwoven cellulose fiber fabric having the above-mentioned properties is used for at least one of the group consisting of a wipe, a dryer sheet, a filter, a hygiene product, a medical application product, a geotextile, agrotextile, clothing, a product for building technology, an automotive product, a furnishing, an industrial product, a product related to beauty, leisure, sports or travel, and a product related to school or office.

According to still another exemplary embodiment, a product or composite is provided which comprises a fabric having the above mentioned properties.

In the context of this application, the term "nonwoven cellulose fiber fabric" (which may also be denoted as nonwoven cellulose filament fabric) may particularly denote a fabric or web composed of a plurality of substantially endless fibers. The term "substantially endless fibers" has in particular the meaning of filament fibers having a significantly longer length than conventional staple fibers. In an alternative formulation, the term "substantially endless fibers" may in particular have the meaning of a web formed of filament fibers having a significantly smaller amount of fiber ends per volume than conventional staple fibers. In particular, endless fibers of a fabric according to an exemplary embodiment of the invention may have an amount of fiber ends per volume of less than 10,000 ends/cm³, in particular less than 5,000 ends/cm³. For instance, when staple fibers are used as a substitute for cotton, they may have a length of 38 mm (corresponding to a typical natural length of cotton fibers). In contrast to this, substantially endless fibers of the nonwoven cellulose fiber fabric may have a length of at least 200 mm, in particular at least 1000 mm. However, a person skilled in the art will be aware of the fact that even endless cellulose fibers may have interruptions, which may be formed by processes during and/or after fiber formation. As a consequence, a nonwoven cellulose fiber fabric made of substantially endless cellulose fibers has a significantly lower number of fibers per mass compared to nonwoven fabric made from staple fibers of the same denier. A nonwoven cellulose fiber fabric may be manufactured by spinning a plurality of fibers and by attenuating and stretching the latter towards a preferably moving fiber support unit. Thereby, a three-dimensional network or web of cellulose fibers is formed, constituting the nonwoven cellulose fiber fabric. The fabric may be made of cellulose as main or only constituent.

In the context of this application, the term "lyocell spinning solution" may particularly denote a solvent (for example a polar solution of a material such as N-methyl-morpholine, NMMO, "amine oxide" or "AO") in which cellulose (for instance wood pulp or other cellulose-based feedstock) is dissolved. The lyocell spinning solution is a solution rather than a melt. Cellulose filaments may be generated from the lyocell spinning solution by reducing the concentration of the solvent, for instance by contacting said filaments with water. The process of initial generation of cellulose fibers from a lyocell spinning solution can be described as coagulation.

In the context of this application, the term "gas flow" may particularly denote a flow of gas such as air substantially parallel to the moving direction of the cellulose fiber or its preform (i.e. lyocell spinning solution) while and/or after the lyocell spinning solution leaves or has left the spinneret.

In the context of this application, the term "coagulation fluid" may particularly denote a non-solvent fluid (i.e. a gas and/or a liquid, optionally including solid particles) which has the capability of diluting the lyocell spinning solution and exchanging with the solvent to such an extent that the cellulose fibers are formed from the lyocell filaments. For instance, such a coagulation fluid may be water mist.

In the context of this application, the term "process parameters" may particularly denote all physical parameters and/or chemical parameters and/or device parameters of substances and/or device components used for manufacturing nonwoven cellulose fiber fabric which may have an impact on the properties of the fibers and/or the fabric, in particular on fiber diameter and/or fiber diameter distribution. Such process parameters may be adjustable automatically by a control unit and/or manually by a user to thereby tune or adjust the properties of the fibers of the nonwoven cellulose fiber fabric. Physical parameters which may have an impact on the properties of the fibers (in particular on their diameter or diameter distribution) may be temperature, pressure and/or density of the various media involved in the process (such as the lyocell spinning solution, the coagulation fluid, the gas flow, etc.). Chemical parameters may be concentration, amount, pH value of involved media (such as the lyocell spinning solution, the coagulation fluid, etc.). Device parameters may be size of and/or distances between orifices, distance between orifices and fiber support unit, speed of transportation of fiber support unit, the provision of one or more optional *in situ* post processing units, the gas flow, etc.

The term "fibers" may particularly denote elongated pieces of a material comprising cellulose, for instance roughly round or non-regularly formed in cross-section, optionally twisted with other fibers. Fibers may have an aspect ratio which is larger than 10, particularly larger than 100, more particularly larger than 1000. The aspect ratio is the ratio between the length of the fiber and a diameter of the fiber. Fibers may form networks by being interconnected by merging (so that an integral multi-fiber structure is formed) or by friction (so that the fibers remain separate but are weakly mechanically coupled by a friction force exerted when mutually moving the fibers being in physical contact with one another). Fibers may have a substantially cylindrical form which may however be straight, bent, kinked, or curved. Fibers may consist of a single homogenous material (i.e. cellulose). However, the fibers may also comprise one or more additives. Liquid materials such as water or oil may be accumulated between the fibers.

In the context of this document, a "jet with orifices" (which may for instance be denoted as an "arrangement of orifices") may be any structure comprising an arrangement of orifices which are linearly arranged.

In the context of this application, the term "pores" may particularly denote miniature openings or even oblong conduits within the fiber network and delimited between fibers. Pores may define channels along which particles (such as dust particles) or liquids may move inside the fabric or out of the fabric. Pores may extend from an exterior of the fabric into an interior thereof to thereby form fluidic channels for particles or other medium (such as a liquid). The pores may be capable of accommodating or storing a medium such as solid or viscous particles or a solid, liquid or viscous active agent.

In the context of this application, the term "cavity" may particularly denote hollow spaces in an interior of the fiber network, being delimited between fibers and being capable of accommodating or storing a medium such as solid or viscous particles or a solid, liquid or viscous agent or preparation. A cavity may be in fluid communication with one or more pores so that a medium to be accommodated in a cavity may be transported to the cavity and/or out of the cavity by flowing or moving along the one or more pores. A cavity may have a larger diameter than a connected pore.

In the context of this application, the term "pore size" may particularly denote a characteristic dimension of a pore indicative of a dimension of particles capable of moving along the pore without getting stuck.

In the context of this application, the term "size range" of a plurality of pores may particularly denote a range between a minimum pore size and a maximum pore size of the respective plurality of set of pores. The size range may also cover modifications of a pore size of one and the same pore in different conditions, for instance in different humidity conditions (in particular in a dry state and in a wet state of the fibers delimiting the pores). For the definition of "dry state" and "wet state" see the standards established in the textile industry, e.g. the BISFA Booklet, edition 2004.

In the context of this application, the term "retaining particles" may particularly denote a functional property of a set of pores or cavities relating to the fact that corresponding particles are retained in an interior of the pores or cavities since channels defined by the pores or cavities are too small to enable the particles to move along the channels. As a consequence, the particles are then retained in an interior of the pore structure. For instance, a particular retaining function can be fulfilled by fibers delimiting corresponding pores or cavities, when the fibers are in a swollen state as a result of moisture in the fabric swelling the fibers.

In the context of this application, the term "releasing particles" may particularly denote a functional property of a set of pores or cavities relating to the fact that corresponding particles are released from an interior of the pores or cavities to an exterior of the fabric since channels defined by the pores or cavities are sufficiently large to enable the particles to move along the channels. As a consequence, the particles are then released out of an interior of the pore structure. For instance, a release function can be fulfilled by fibers delimiting corresponding pores, which fibers are in a shrunk or non-swollen state as a result of a dry state of the fiber, i.e. the absence of moisture or in the presence of only a minor amount of moisture in the fabric swelling the fibers.

In the context of this application, the term "adjusting a condition of the fabric" may particularly denote setting one or more physical and/or chemical parameters of the fabric to a certain value to thereby modify the fabric's capability of retaining or releasing an active agent accommodated in the pores between the fibers. Adjusting such a condition may change an average fiber-fiber distance, may change a fiber holding force (in particular a capillary force) indicating a force by which a fiber or a set of fibers can hold certain liquids such as an active agent, etc. For instance, the condition may be a degree of humidity of the fabric or the fibers thereof. Other conditions are a mechanical tension state or a temperature of the fabric.

In the context of this application, the term "humidity state of the fibers" may particularly denote an amount of moisture (in particular water or another aqueous or non-aqueous liquid) stored in an interior of the fiber material. In other words, the humidity state of a fiber may indicate which mass of liquid has been soaked by a fiber, in particular related to the dry fiber mass. Even more specifically, a dry fiber is in another humidity state than a wet fiber.

In the context of this application, the term "active agent" may particularly denote a substance that may produce a chemical reaction or may have a physical impact, that may have, in turn, an impact on the physical (for instance mechanical, electrical, magnetic, optical, etc.) properties of the fabric or an environment thereof, and/or that may have a biological impact (for instance a medical impact, so that the active agent may for instance be a pharmaceutically active agent). The active agent may comprise or consist of one or more solid particles and/or one or more liquids or viscous substances.

According to an exemplary embodiment of a first aspect of the invention, a nonwoven cellulose fiber fabric is provided which comprises different groups of pores delimited between fibers of the fabric, wherein the different groups have assigned different (in particular overlapping or non-overlapping) size ranges. Thus, the different groups of pores defined by the different pore size ranges may have different properties in terms of an interaction of the fabric with another medium. Such another medium may for instance be particles which shall be transferred into the interior of the fabric (for instance dust, when the fabric is used as a cleaning wipe) and/or may be a medium (for instance a liquid) which has been stored in the fabric and shall be released to an environment (for instance a medication accommodated in the pores to be released when the fabric is administered to a patient) in a controlled way. According to an exemplary embodiment of the invention, it has turned out that a corresponding pore structure of nonwoven cellulose fiber fabric can be manufactured (in particular by a manufacture directly from a lyocell spinning solution) with highly predictable properties in terms of retaining and releasing a medium. Such pore properties may be adjusted by adjusting process parameters of a method of manufacturing such a nonwoven cellulose fiber fabric. For instance, adjustment of fiber diameter and/or fiber diameter distribution, integrally formed merging positions between fibers, formation of a multilayer fabric with individually controllable properties of the individual but nevertheless interconnected fiber layers, post processing (for instance by hydro-entanglement), etc. may be used as process parameters usable for adjusting pore properties and for defining different pore groups of different sizes.

Descriptively speaking, different pore structures in form of the primary pores and the secondary pores may be formed in a fabric according to an exemplary embodiment of the invention. The primary pores may be formed for instance as gaps between the individual fibers of the fiber fabric. The size of the primary pores can be adjusted, for instance by adjusting fiber diameter, fiber diameter variation, merging, etc. The secondary pores can for instance be manufactured by hydroentanglement or needle punching of the fiber network or fabric. These differently sized primary pores and secondary pores can be used for accommodating particles of different dimensions or sizes within the nonwoven cellulose fiber fabric.

According to an exemplary embodiment of a second aspect of the invention (which can be combined with the above mentioned first aspect or which may be realized independently of the first aspect), a nonwoven cellulose fiber fabric is provided which comprises a pore structure accommodating and normally retaining an active agent. The fabric may be advantageously further configured so that, by adjusting a condition of the fabric, the previously retained active agent may be released to an environment in a defined way. For instance, adjustment of fiber diameter and/or fiber diameter distribution, integrally formed merging positions between fibers, formation of multilayer fabric with individually controllable properties of the individual but nevertheless interconnected fiber layers, post processing (for instance by hydro-entanglement), etc. may be used as process parameters usable for adjusting pore properties allowing for precisely defining conditions under which release of the active agent is initiated. For instance, such a condition triggering release of the active agent is a certain humidity state of the fibers, a temperature of the fibers, a mechanical tension of the fibers (for instance exerted by a user pulling or squeezing on the fabric or bending the fabric), etc. Thus, nonwoven cellulose fiber fabric may be provided with a defined and predictable active agent release characteristics.

### Detailed description of embodiments of the invention

In the following, further exemplary embodiments of the nonwoven cellulose fiber fabric, the method of manufacturing a nonwoven cellulose fiber fabric, the device for manufacturing a nonwoven cellulose fiber fabric, the method of controlling release of an active agent, the product or composite, and the method of use are described.

In an embodiment, at least part of the fibers form part of both the first plurality of the fibers and the second plurality of the fibers. In other words, one and the same fiber may be used for delimiting a primary pore and a secondary pore. Primary pores and secondary pores may therefore be present in the same fabric portion of the fabric. Additionally or alternatively, at least part of the fibers form part of only the first plurality of the fibers (delimiting the first pores) or only the second plurality of the fibers (delimiting the second pores). Thus, first fibers may delimit only first pores, and other second fibers may delimit only second pores.

In an embodiment, a cellulose type endless fiber fabric with several types of pores is provided, wherein the fabric comprises first or primary pores enabling a cavity volume dependent insertion and removal of particles and comprises second or secondary pores enabling a swelling and/or shrinking supported particle retaining and/or release function. For both the primary pores and the secondary pores, swelling and shrinking or insertion and removal of particles may occur, depending on the size of the particles in relation to the size of the pores.

In an embodiment, the fabric comprises a first fabric portion having the plurality of first pores, and comprises a second fabric portion being different from the first fabric portion and having the plurality of first pores in a different size. In such an embodiment, the set of first pores is located in another fabric portion (for instance in a certain distinguishable layer of the fabric) than the set of first pores with different size being located in another fabric portion (for instance in another distinguishable layer of the fabric). The process parameters for obtaining corresponding pore sizes can be adjusted individually for the different fabric portions, in particular different fabric layers. One example of such an embodiment is a double layer fabric having a first layer with the first set of pores which may serve for instance as a wiping side of the fabric, whereas an opposing second layer of the fabric may for instance serve as an active agent reservoir (for instance retaining a liquid cleaning agent) capable of releasing the active agent by adjusting the properties (for instance the degree of humidity) of the fabric. For instance, when the fabric becomes wet, the active agent may be released.

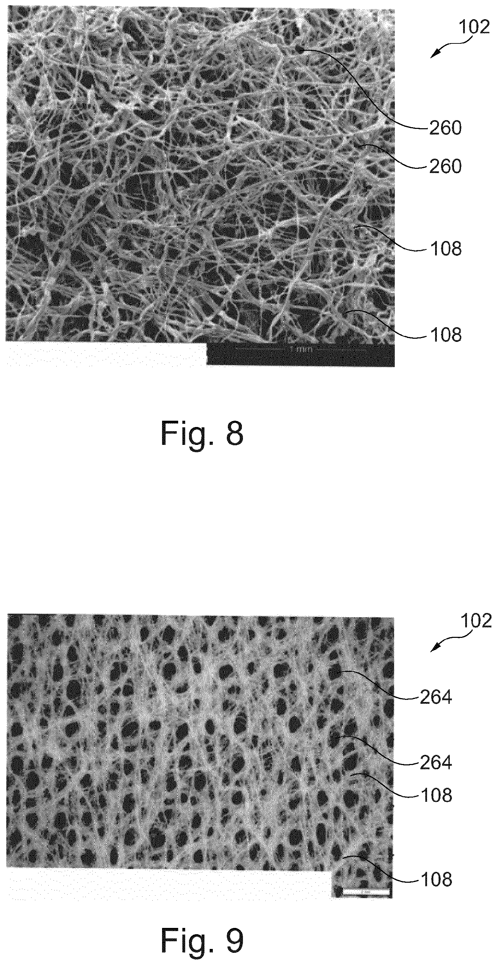

In an embodiment, the first fabric portion has a substantially homogeneous fiber distribution (see Figure 8). In particular, the first fabric portion may be a fiber network as obtained from extruded lyocell spinning solution without the need of further processing the first fabric portion. In an embodiment, the second fabric portion has a non-homogeneous fiber distribution (see Figure 9). In particular, the second portion may be a fiber network as obtained from extruded lyocell spinning solution followed by further processing (for instance hydro-entangling) the second fabric portion for forming the inhomogeneity.

In an embodiment, the first size range and the second size range have no size in common. For example, the first range may range from a first lower size value up to a first upper size value, whereas the second range may range from a second lower size value up to a second upper size value. The first upper size value may be lower than the second lower size value. Consequently, the pore sizes of the first size range and the second size range may be free of an overlap. This ensures that the different functionalities of different fiber portions assigned to different size ranges may be clearly separated. However, it is alternatively possible that the smaller first pores and the larger second pores are uniformly distributed over the fabric without distinguishing different fabric portions with different pore size ranges. The first pores and the second pores may have different average diameters.

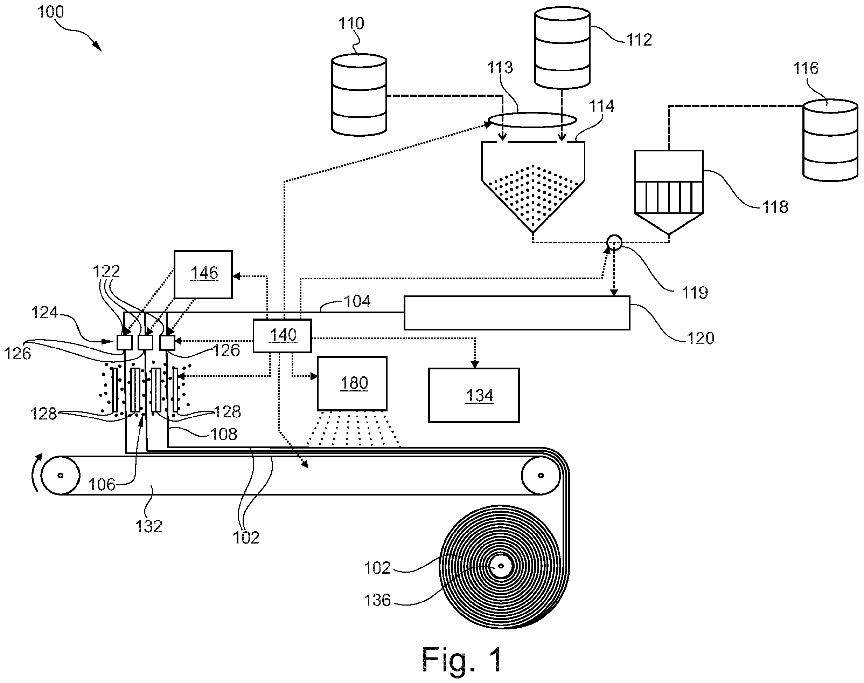

In an embodiment, the fibers are configured so that at least one of the plurality of first pores and the plurality of second pores modify the respective size range by at least one of the group consisting of swelling and shrinking depending on a humidity state of the fibers. The cellulose fibers manufactured directly from lyocell spinning solution (compare for instance the manufacturing method described below referring to Figure 1) may have an intrinsic property of accommodating moisture (for instance water) within an interior of the respective fiber. This is in particular the case when the fibers comprise or consists of microfibrillar cellulose. Such a microfibril may be denoted as a very fine fibril, or fiber-like strand, consisting of cellulose. Cellulose fibers may be built up of fiber bundles, which may be composed of smaller elements called microfibrils which can also be in the submicron range. Through a fibrillation process, the cellulose fibers may be converted into a three-dimensional network of microfibrils with a high surface area. Thus, the fabricated cellulose fibers themselves may have the capability of soaking water or other moisture. In the presence of moisture, the fibers will therefore swell and will consequently increase their dimensions, whereas in the absence of moisture, the fibers will shrink and will consequently decrease their dimensions. As a result, the amount of moisture available in an environment of the fabric will define the size of cavities within the fiber network. This, in turn, has an impact on the capillary forces within the fabric being a physical mechanism of retaining a medium (such as particles, an active agent, liquid, etc.) within the fiber network. Therefore, controlling moisture is a simple and efficient mechanism of controlling medium retaining and medium releasing properties of the fabric and portions thereof.

In an embodiment, the fabric is configured so that the first particles are enabled to enter into or leave the first pores. The fabric comprising the first particles in an interior thereof will therefore allow the first particles to move between an interior and an exterior of the fabric through channels defined between the first pores when the dimension of the first particles is smaller than the smallest dimensions of a channel from an exterior of the fabric to an interior accommodation position. For instance, the fabric may be configured so that dust particles (having characteristic sizes) are enabled to enter the first pores, which may be for instance advantageous for applications such as a wipe.

In an embodiment, the first size range of the first pores is configured so that first particles with a diameter in a range between 0.5 µm and 500 µm, in particular in a range between 3 µm and 300 µm are enabled to enter into or leave the first pores in a dry state of the fibers. Thus, the first pores may have a diameter in a range between 0.5 µm and 500 µm, in particular in a range between 3 µm and 300 µm, in a dry state of the fibers. Additionally or alternatively, the first size range of the first pores may be configured so that first particles with a diameter in a range between 0.5 µm and 500 µm, in particular in a range between 3 µm and 300 µm, are disabled to enter into or leave the first pores in a wet state of the fibers. In a dry state of the fibers, no or only minor amounts of liquids are accommodated within the fibers, which are then present in a shrunk state. Consequently, channels defined between the fibers may be large and a motion of the first particles between an interior and an exterior of the fabric can be enabled. In a wet or soaked state of the fibers however, a significant amount of liquid is accommodated within the fibers, which are then present in a swollen state. Consequently, channels defined between the fibers may be smaller and a motion of the first particles between an interior and an exterior of the fabric can be disabled. Moisture control of the fabric may therefore be used as a simple mechanism of adjusting a range of particle sizes capable of entering or leaving the first pores.

Correspondingly, the fabric may be configured so that second particles are enabled to selectively enter into or leave the second pores. For instance, the second size range of the second pores may be configured so that second particles with a diameter in a range in a range between 0.1 mm and 5 mm, in particular in a range between 0.2 mm and 2 mm, are enabled to enter into or leave the second pores in a dry state of the fibers. Accordingly, the second size range of the second pores may be configured so that second particles with a diameter in a range between 0.1 mm and 5 mm, in particular in a range between 0.2 mm and 2 mm, are disabled to enter into or leave the second pores in a wet state of the fibers. The second pores may have a diameter in a range between 0.1 mm and 5 mm, in particular in a range between 0.2 mm and 2 mm, in a dry state of the fibers.

In an embodiment, the fabric (or a product comprising the fabric) comprises a medium filling at least one of the plurality of first pores and the plurality of second pores. In particular, the fabric (of the product) may comprise at least 1 mass percent of the medium, more particularly at least 10 mass percent of the medium (i.e. a ratio between a mass of the medium only and the entire mass of the fabric including the medium may be at least 1% or even at least 10%). The medium may be a liquid medium and/or a solid medium (such as particles). Such a medium may comprise or consist of an active agent.

In an embodiment, the fabric comprises an active agent (for instance as the second particles) accommodated in the plurality of second pores. The active agent accommodated within the second pores, for instance supported by capillary forces within the fiber network, may be retained within the fabric in a first fabric condition (for instance related to a certain value of humidity, temperature, pressure, mechanical load exerted to the fabric, etc.) but may be released from the fabric to an environment in a second fabric condition (for instance related to another value of humidity, temperature, pressure, mechanical load exerted to the fabric, etc.). Active agent release may therefore be precisely controlled for a product based on a nonwoven cellulose fiber fabric according to an exemplary embodiment of the invention.

In an embodiment, the endless fibers have an amount of fiber ends per volume of less than 10,000 ends/cm³, in particular less than 5,000 ends/cm³ in a fabric having a density of 0.1 t/m³ Since the fibers of the nonwoven cellulose fiber fabric according to an exemplary embodiment of the invention are endless fibers, the number of (in practice not completely unavoidable, as known by a skilled person) fiber ends of the fabric may be very small. In contrast to this, fabrics comprising conventional staple fibers may have significantly higher numbers of fiber ends per volume. It has turned out that precision, predictability and reproducibility of the medium retaining and/or medium releasing and/or medium passing properties of the fabric can be adjusted better or more precisely within the nonwoven fabric comprising substantially endless fibers in comparison to nonwoven fabrics comprising staple fibers. Therefore, the use of endless fibers with very small amount of free ends is particularly appropriate for accurately defining medium interaction properties of the fabric.

In an embodiment, the fibers differ (in particular in a certain moisture state, for instance a dry state of the fibers) concerning fiber diameter so that a ratio between an average diameter of the 10% thinnest fibers and an average diameter of the 10% thickest fibers is more than 0.01, in particular is more than 0.05, more particularly is more than 0.1. In particular, different fibers (for instance related to different fiber portions corresponding, in turn, to the differently sized groups of pores) may differ concerning fiber diameter. Additionally or alternatively, it is also possible that one and the same fiber has different sections of different diameters. For instance, a ratio between a largest fiber diameter and a smallest fiber diameter may be more than 1.5 (or more than 2.5, or even more than 4). These values may particularly denote that a ratio between largest fiber diameter and smallest fiber diameter, multiplied by 100%, wherein 100% is subtracted from the obtained result, gives a value above 50% (or 150% or 300%, respectively). Inhomogeneously adjusting fiber diameters has turned out as an efficient mechanism of influencing medium retaining and/or medium releasing properties of the fiber fabric, since fiber dimensions have an impact on fluid interaction properties of the fabric such as capillary forces, definition of cavity shapes, etc.

In an embodiment, at least 80 mass percent of the fibers have an average fiber diameter in a range between 1 µm and 40 µm, in particular between 3 µm and 15 µm. By the described method and when adjusting the process parameters accordingly, also fibers with very small dimensions (also in a range between 1 µm and 5 µm, or below) may be formed. With such small fibers, a fabric with a smooth surface may be formed which is nevertheless rigid as a whole.

In an embodiment, the fabric is configured so that a wicking speed is at least 0. 25 g water/g fabric/s. More particularly, the wicking speed may be at least 0.4 g water/g fabric/s, in particular at least 0.5 g water/g fabric/s. The wicking speed may correspond to the velocity according to which a medium is soaked from an exterior of the fabric into an interior thereof. By correspondingly adjusting the process parameters of the method of manufacturing nonwoven cellulose fiber fabric according to an exemplary embodiment of the invention, it is possible to obtain a fabric soaking medium (in particular moisture) very quickly. Also a liquid spreading speed may be correspondingly high. This may be highly advantageous for certain applications such as wipes or active agent release fabric.

The values for the wicking speed described in this document relate to a "wicking speed test" wherein the sample under test (i.e. the respective fabric) is conditioned in a fully dry state. Fully dry means that after manufacturing the fabric (which includes a drying) the fabric has been conditioned for 24 hours at a standard climate being defined with a temperature of 23°C ± 2°C, with a relative humidity of 50% ± 5%. All measurement, unless notified otherwise, have been performed under this standard climate. In the "wicking speed test" the sample under test is placed onto a test table. At its center, the test table is connected via an opening and a channel with a liquid reservoir. The liquid reservoir is filled with fully distilled water. The height of the test table corresponds exactly to the filling level of the water within the liquid reservoir. Thereby, it is ensured that no hydrostatic pressure is present and the suction respectively the wicking of the sample under test is generated exclusively by the suction power of the sample under test. During the actual "wicking speed test" the volume of the water, which is absorbed by the sample under test, is continuously refilled into the liquid reservoir with a syringe. This means that the level of the liquid is always kept constant. The volume of the refilled water is converted into the mass of the refilled water (via the known density of mass of distilled water). It is obvious that with this procedure the wicking speed decreases with time because the suction power of the sample under test decreases with an increasing "absorption load" of the sample under test caused by the absorbed water. The procedure of refilling is continued until for the refilling of water a threshold value of 0.005g per 20 seconds is reached. A measurement curve depicting the mass of added water as a function of time is recorded and evaluated. In this document the wicking speed is the slope of this measurement curve with a first time slot of 10 seconds starting from the beginning of the actual test.

In an embodiment, the fabric is configured so that pores and/or cavities defined between at least part of the pores undergo a diameter change of at least 20%, in particular at least 30%, between a dry state and a wet state of the fabric. In other embodiments, the mentioned percentages may be smaller, for instance in a range between 1% and 5%. The diameter reduction of the cavity may be calculated as 100% minus the ratio between the smaller diameter in swollen state and the larger diameter in dry state multiplied by 100% and may be given in percent (for instance 100% - 75 µm/100 µm*100%= 25%, when the smaller diameter is 75 µm and the larger diameter is 100 µm). Since corresponding fiber dimensions can be changed over a broad range by controlling the humidity conditions of the fabric, also the size of the pores and correspondingly cavities defined between the fibers may be simply adjusted over a broad range. Therefore, the addition or removal of moisture or the like may be used as a switching mechanism for switching the fabric between a particle permeable state and a particle impermeable state as a result of the adjustable diameter variation.

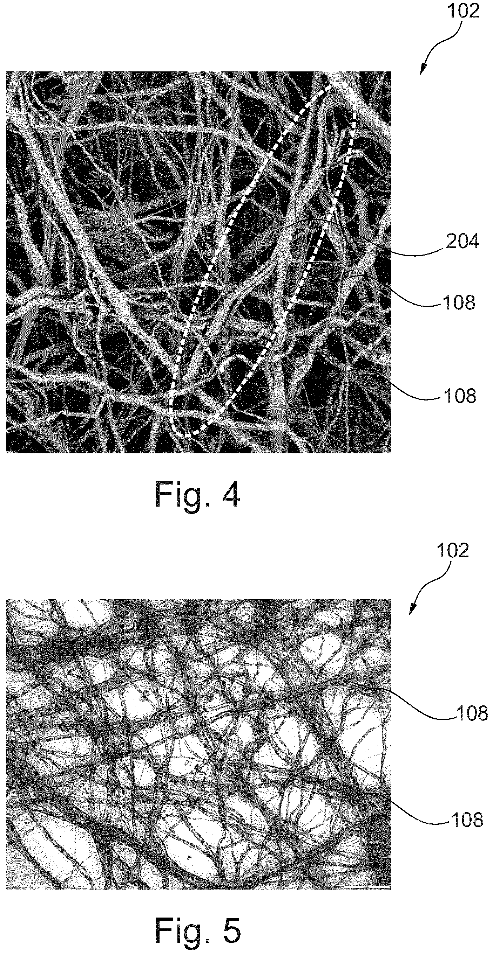

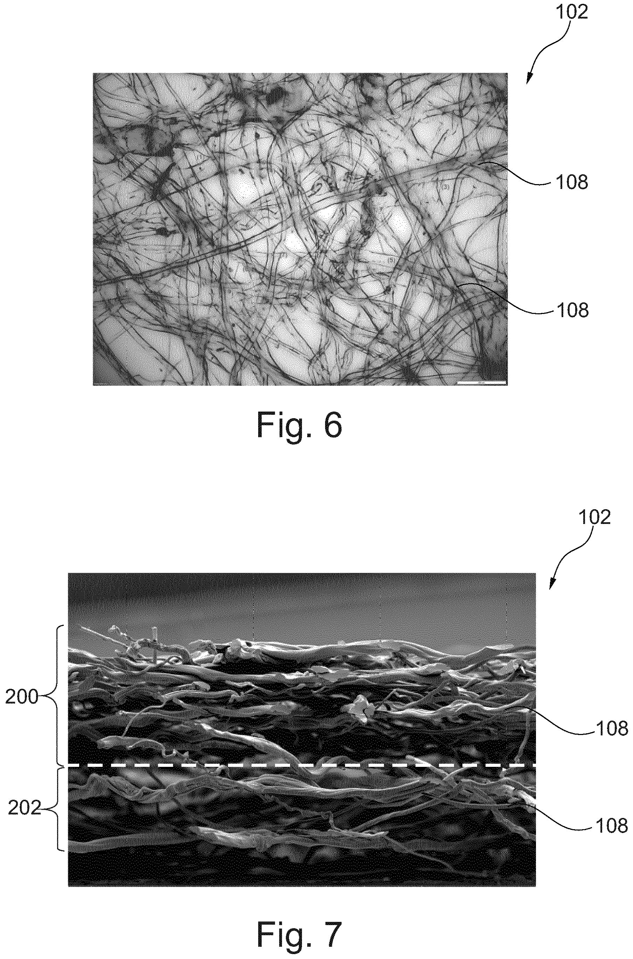

For quantifying the swelling behavior of the fibers in the presence of moisture such as water, a sample fabric may be prepared. For instance, two or three pieces of the fabric may be cut out, each having an area of 1 cm². The individual pieces of fabric may then be transferred without change of their structure onto an object carrier of a microscope. A zoom factor of 10 may be adjusted with the microscope (which may be an Olympus BX 51 microscope). The microscope may be operated in a black and white mode. Firstly, the fabric samples may be measured in a dry state by manually creating image sequences starting from a lowermost plane of the sample up to an uppermost plane. Thereafter, the same portion of the sample is used and humidified by supplying a droplet of water. In view of capillary forces, the water will be distributed over the sample volume. After 1 minute, image sequences of the humidified sample may be captured, using the procedure described above for the dry sample. A decrease of the volumes between the fibers could be identified. Reference is made to a comparison of Figure 5 and Figure 6 described below.

In a corresponding investigation of a nonwoven cellulose fiber fabric according to an exemplary embodiment of the invention, the following size distribution of the length of the cavities in micrometers has been obtained:

| Number | Dry fabric | Wet fabric |
|---|---|---|
| 1 | 114,38 µm | 49,73 µm |
| 2 | 55,60 µm | 11,74 µm |
| 3 | 59,23 µm | 17,73 µm |
| 4 | 178,83 µm | 113,02 µm |
| 5 | 83,48 µm | 38,88 µm |
| 6 | 176,77 µm | 59,91 µm |
| 7 | 64,03 µm | 25,16 µm |
| 8 | 73,53 µm | 49,40 µm |
| 9 | 67,11 µm | 26,85 µm |
| 10 | 98,73 µm | 50,54 µm |

In an embodiment, at least part of (in particular at least 10%, more particularly at least 20% of) the fibers are integrally merged at merging positions. In the context of this application, the term "merging" may particularly denote an integral interconnection of different fibers at the respective merging position which results in the formation of one integrally connected fiber structure composed of the previously separate fiber preforms. Merging may be denoted as a fiber-fiber connection being established during coagulation of one, some or all of the merged fibers. Interconnected fibers may strongly adhere to one another at a respective merging position without a different additional material (such as a separate adhesive) so as to form a common structure. Separation of merged fibers may require destruction of the fiber network or part thereof. According to the described embodiment, a nonwoven cellulose fiber fabric is provided in which some or all of the fibers are integrally connected to one another by merging. Merging may be triggered by a corresponding control of the process parameters of a method of manufacturing the nonwoven cellulose fiber fabric. In particular, coagulation of filaments of lyocell spinning solution may be triggered (or at least completed) after the first contact between these filaments being not yet in the precipitated solid fiber state. Thereby, interaction between these filaments while still being in the solution phase and then or thereafter converting them into the solid phase by coagulation allows to properly adjust the merging characteristics. A degree of merging is a powerful parameter which can be used for fine-tuning the properties of the manufactured fabric. In particular, mechanical stability of the network is the larger the higher the density of merging positions is. By an inhomogeneous distribution of merging positions over the volume of the fabric, it is also possible to adjust regions of high mechanical stability and other regions of low mechanical stability. For instance, separation of the fabric into separate parts can be precisely defined to happen locally at mechanical weak regions with a low number of merging positions. In a preferred embodiment, merging between fibers is triggered by bringing different fiber preforms in form of lyocell spinning solution in direct contact with one another prior to coagulation. By such a coagulation process, single material common precipitation of the fibers is executed, thereby forming the merging positions.

It has been found by the present inventors that adjusting the merging properties between fibers is also a powerful tool of influencing the medium retaining and medium releasing properties of the fabric, since the formation of merging positions has an impact on the pore geometry, capillary forces, cavities sizes, expansion capability of the fabric in the presence of moisture, etc.

In an embodiment, the merging points or merging positions consist of the same material as the merged fibers. Thus, the merging positions may be formed by cellulose material resulting directly from the coagulation of lyocell spinning solution. This not only renders the separate provision of a fiber connection material (such as an adhesive or a binder) dispensable, but also keeps the fabric clean and made of a single material. Formation of fibers having a non-circular cross-section and formation of fibers being interconnected by merging can be done by one single common process, and therefore with low effort. The reason for this is that both the formation of merging positions (such as merging points, merging pads of merging lines) between fibers and the formation of fibers having a cross-section deviating from a perfectly circular diameter can be carried out by exerting a mechanical force on filaments of lyocell spinning solution prior to the completion of coagulation. Nevertheless, filaments of lyocell spinning solution may be influenced mechanically while they are still in a liquid phase.

In an embodiment, the method further comprises adding moisture to at least a portion of the fabric to thereby decrease at least one of the size ranges by moisture-based swelling of at least part of the fibers. Correspondingly, the method may further comprise removing moisture from at least a portion of the fabric to thereby increase at least one of the size ranges by moisture-based shrinkage of at least part of the fibers. Adjustment of the humidity state of the fabric may therefore be used as a control parameter for controlling medium retaining and/or medium releasing and/or medium passing (or blocking) properties of the fabric or parts thereof.

More generally, the method may further comprise adjusting a humidity state of the fibers for modifying the respective size range of at least one of the group consisting of the plurality of first pores and the plurality of second pores. Changing the humidity state may change fiber diameter, void volume within the fiber network, the mechanical tension properties of the fiber network, etc. and may therefore have an impact on the pore sizes.

In an embodiment, each of the plurality of first pores and/or cavities in fluid communication with at least part of the first pores is delimited between at least three fibers of the fabric. For instance, a droplet of liquid may be held, by the influence of capillary forces, electric forces (i.e. forces resulting from electric charge or multipoles), van der Waals forces, etc. between three or more fibers defining or delimiting a cavity or pore. Also merging positions between these fibers may contribute to the definition of the cavity volume or pore geometry. Thus, adjustment of the process parameters of manufacturing the fiber fabric in terms of adjusting an average fiber-fiber distance, fiber diameter, merging properties between fibers, etc. allows to precisely determine cavity properties within the fabric. A cavity (or coupled cavity) may be denoted as a volumetric unit enclosed by at least three fibers.

In an embodiment, each of the plurality of enlarged second pores and/or cavities in fluid communication with at least part of the second pores is formed by hydro-entanglement. It may use fine, high pressure jets of water which penetrate the fabric, hit the conveyor belt of the fiber support unit and bounce back causing the fibers to entangle. Thereby, pores of significant size may be formed (compare for instance Figure 9). Selective hydro-entanglement of only a portion of the fabric (for instance of only one layer of a multilayer fabric) allows to form corresponding large second pores only in a precisely definable fabric portion. This is possible when a hydroentangling unit is located between two jets, each forming a layer of nonwoven fabric. By taking this measure, it is for instance possible to distinguish a fabric portion having just primary pores and another fabric portion also having the secondary pores.

In an embodiment, the method further comprises providing a further active agent in at least part of the pores and/or the cavities, and adjusting a condition of the fabric (in particular a humidity state of the fibers) for triggering release of the further active agent out of the pores and/or the cavities after having completed the release of the active agent out of the pores and/or the cavities. In particular, such a procedure may involve providing a further active agent in at least part of the pores, and adjusting a humidity state of the fibers for triggering one of the group consisting of a swelling and a shrinkage of the fibers to thereby control release of the further active agent out of the pores after having completed the release of the active agent out of the pores. By adjusting a condition of the fabric (such as a temperature, pressure, a mechanical force exerted to the fabric or a portion thereof, the humidity state, etc.) the fabric may be impacted so that, firstly, only the first active agent is released. When the pores accommodating the first active agent and other pores accommodating the second active agent differ concerning size, diameter of pore delimiting fibers, degree of merging, etc., it is possible to precisely adjust the order according to which the respective active agent is released from the respective pores.

More specifically, a nonwoven cellulose fiber fabric according to an exemplary embodiment of the invention may be used for manufacturing a fabric product comprising multiple active agents. When getting in contact with an appropriate liquid such as water, the individual active agents may be released temporarily subsequently, but not simultaneously. The active agents may be accommodated in pores or cavities of the endless fiber fabric. Release of one or both of the active agents may be triggered by swelling or shrinking of fibers (or in other words, may be triggered when the degree of humidity of the fabric exceeds or falls below an active agent specific threshold value). The timing characteristic of the multiple active agents release can be controlled by correspondingly controlling the process parameters of manufacturing the fabric. For instance, a predictable sequence of active agents release may be controlled to occur when a piece of fabric including the active agents is administered as a medication to a patient and enters a human body with its characteristic humidity properties. Such fabric may be used for manufacturing a product such as a facial mask, bandaging material, products accommodating cosmetic or therapeutic active agents, wipes, dryer sheets, etc.

In another embodiment, release of two or more different active agents accommodated in different pores and/or cavities of the fabric need not be necessarily free of a temporal overlap (as in the above described embodiment). It is for instance also possible that the release of two different active agents is controlled (for instance by controlling a moisture level of the fabric) to occur simultaneously or temporally spaced with a certain temporal overlap.

In an embodiment, the fibers have a copper content of less than 5 ppm and/or have a nickel content of less than 2 ppm. The ppm values mentioned in this application all relate to mass (rather than to volume). Apart from this, the heavy metal contamination of the fibers or the fabric may be not more than 10 ppm for each individual chemical element. Due to the use of a lyocell spinning solution as a basis for the formation of the endless fiber-based fabric (in particular when involving a solvent such as N-methyl-morpholine, NMMO), the contamination of the fabric with heavy metals such as copper or nickel (which may cause allergic reactions of a user) may be kept extremely small. Due to the concept of direct merging under certain conditions adjustable by process control, no extra material (such as a glue or the like) needs to be introduced in the process for interconnecting the fibers. This keeps contaminations of the fabric very low.

In an embodiment, the first plurality of pores and the second plurality of pores are located in different distinguishable (i.e. showing a visible separation or interface region in between the layers) layers. More specifically, fibers of different layers are integrally merged at at least one merging position between the layers. Hence, different ones of the fibers being located at least partially in different distinguishable layers (which may be identical or which may differ concerning one or more parameters such as merging factor, average fiber diameter, etc.) may be integrally connected at at least one merging position. For instance, two (or more) different layers of a fabric may be formed by serially aligning two (or more) jets with orifices through which lyocell spinning solution is extruded for coagulation and fiber formation. When such an arrangement is combined with a moving fiber support unit (such as a conveyor belt with a fiber accommodation surface), a first layer of fibers is formed on the fiber support unit by the first jet, and the second jet forms a second layer of fibers on the first layer when the moving fiber support unit reaches the position of the second jet. The process parameters of this method may be adjusted so that merging points are formed between the first layer and the second layer. In particular, fibers of the second layer under formation being not yet fully cured or solidified by coagulation may for example still have exterior skin or surface regions which are still in the liquid lyocell solution phase and not yet in the fully cured solid state. When such pre-fiber structures come into contact with one another and fully cure into the solid fiber state thereafter, this may result in the formation of two merged fibers at an interface between different layers. The higher the number of merging positions, the higher is the stability of the interconnection between the layers of the fabric. Thus, controlling merging allows to control rigidity of the connection between the layers of the fabric. Merging can be controlled, for example, by adjusting the degree of curing or coagulation before pre-fiber structures of a respective layer reach the fiber support plate on an underlying layer of fibers or pre-fiber structures. By merging of fibers of different layers at an interface there between, undesired separation of the layers may be prevented. In the absence of merging points between the layers, peeling off one layer from the other layer of fibers may be made possible.

In an embodiment, the method further comprises further processing the fibers and/or the fabric after collection on the fiber support unit but preferably still *in situ* with the formation of the nonwoven cellulose fiber fabric with endless fibers. Such *in situ* processes may be those processes being carried out before the manufactured (in particular substantially endless) fabric is stored (for instance wound by a winder) for shipping to a product manufacture destination. For instance, such a further processing or post processing may involve hydro-entanglement. Hydro-entanglement may be denoted as a bonding process for wet or dry fibrous webs, the resulting bonded fabric being a nonwoven. Hydro-entanglement may use fine, high pressure jets of water which penetrate the web, hit a fiber support unit (in particular a conveyor belt) and bounce back causing the fibers to entangle. A corresponding compression of the fabric may render the fabric more compact and mechanically more stable. Additionally or alternatively to hydro-entanglement, steam treatment of the fibers with a pressurized steam may be carried out. Additionally or alternatively, such a further processing or post processing may involve a needling treatment of the manufactured fabric. A needle punching system may be used to bond the fibers of the fabric or web. Needle punched fabrics may be produced when barbed needles are pushed through the fibrous web forcing some fibers through the web, where they remain when the needles are withdrawn. If sufficient fibers are suitably displaced the web may be converted into a fabric by the consolidating effect of these fibers plugs. Yet another further processing or post processing treatment of the web or fabric is an impregnating treatment. Impregnating the network of endless fibers may involve the application of one or more chemicals (such as a softening agent, a hydrophobic agent, and antistatic agent, etc.) on the fabric. Still another further processing treatment of the fabric is calendering. Calendering may be denoted as a finishing process for treating the fabric and may employ a calender to smooth, coat, and/or compress the fabric.

A nonwoven cellulose fiber fabric according to an exemplary embodiment of the invention may also be combined (for instance *in situ* or in a subsequent process) with one or more other materials, to thereby form a composite according to an exemplary embodiment of the invention. Exemplary materials, which can be combined with the fabric for forming such a composite may be selected from a group of materials comprising, but not being limited to, the following materials or combinations thereof: fluff pulp, a fiber suspension, a wetlaid nonwoven, an airlaid nonwoven, a spunbond web, a meltblown web, a carded spunlaced or needlepunched web or other sheet like structures made of various materials. In an embodiment, the connection between the different materials can be done by (but not limited to) one or a combination of the following processes: merging, hydroentanglement, needle punching, hydrogen bonding, thermobonding, gluing by a binder, laminating, and/or calendering.

In the following, exemplary advantageous products comprising, or uses of, a nonwoven cellulose fiber fabric according to exemplary embodiments of the invention are summarized:
Particular uses of the webs, either 100% cellulose fiber webs, or for example webs comprising or consisting of two or more fibers, or chemically modified fibers or fibers with incorporated materials such as anti-bacterial materials, ion exchange materials, active carbon, nano particles, lotions, medical agents or fire retardants, or bicomponent fibers may be as follows:
   The nonwoven cellulose fiber fabric according to exemplary embodiments of the invention may be used for manufacturing wipes such as baby, kitchen, wet wipes, cosmetic, hygiene, medical, cleaning, polishing (car, furniture), dust, industrial, duster and mops wipes.

It is also possible that the nonwoven cellulose fiber fabric according to exemplary embodiments of the invention is used for manufacturing a filter. For instance, such a filter may be an air filter, a HVAC, air condition filter, flue gas filter, liquid filters, coffee filters, tea bags, coffee bags, food filters, water purification filter, blood filter, cigarette filter; cabin filters, oil filters, cartridge filter, vacuum filter, vacuum cleaner bag, dust filter, hydraulic filter, kitchen filter, fan filter, moisture exchange filters, pollen filter, HEVAC/HEPA/ULPA filters, beer filter, milk filter, liquid coolant filter and fruit juices filters.

In yet another embodiment, the nonwoven cellulose fiber fabric may be used for manufacturing absorbent hygiene products. Examples thereof are an acquisition layer, a coverstock, a distribution layer, an absorbent cover, sanitary pads, topsheets, backsheets, leg cuffs, flushable products, pads, nursing pads, disposal underwear, training pants, face masks, beauty facial masks, cosmetic removal pads, washcloths, diapers, and sheets for a laundry dryer releasing an active component (such as a textile softener).

In still another embodiment, the nonwoven cellulose fiber fabric may be used for manufacturing a medical application product. For instance, such medical application products may be disposable caps, gowns, masks and shoe cover, wound care products, sterile packaging products, coverstock products, dressing materials, one way clothing, dialyses products, nasal strips, adhesives for dental plates, disposal underwear, drapes, wraps and packs, sponges, dressings and wipes, bed linen, transdermal drug delivery, shrouds, underpads, procedure packs, heat packs, ostomy bag liners, fixation tapes and incubator mattresses.

In yet another embodiment, the nonwoven cellulose fiber fabric may be used for manufacturing geotextiles. This may involve the production of crop protection covers, capillary matting, water purification, irrigation control, asphalt overlay, soil stabilisation, drainage, sedimentation and erosion control, pond liners, impregnation based, drainage channel liners, ground stabilisation, pit linings, seed blankets, weed control fabrics, greenhouse shading, root bags and biodegradable plant pots. It is also possible to use the nonwoven cellulose fiber fabric for a plant foil (for instance providing a light protection and/or a mechanical protection for a plant, and/or providing the plant or soil with dung or seed).

In another embodiment, the nonwoven cellulose fiber fabric may be used for manufacturing clothing. For example, interlinings, clothing insulation and protection, handbag components, shoe components, belt liners, industrial headwear/foodwear, disposable workwear, clothing and shoe bags and thermal insulation may be manufactured on the basis of such fabric.

In still another embodiment, the nonwoven cellulose fiber fabric may be used for manufacturing products used for building technology. For instance, roofing and tile underlay, underslating, thermal and noise insulation, house wrap, facings for plaster board, pipe wrap, concrete moulding layers, foundations and ground stabilisation, vertical drainages, shingles, roofing felts, noise abatement, reinforcement, sealing material, and damping material (mechanical) may be manufactured using such fabric.

In still another embodiment, the nonwoven cellulose fiber fabric may be used for manufacturing an automotive product. Examples are a cabin filter, boot liners, parcel shelves, heat shields, shelf trim, moulded bonnet liners, boot floor covering, oil filter, headliners, rear parcel shelves, decorative fabrics, airbags, silencer pads, insulation materials, car covers, underpadding, car mats, tapes, backing and tufted carpets, seat covers, door trim, needled carpet, and auto carpet backing.

Still another field of application of fabric manufactured according to exemplary embodiments of the invention are furnishings, such as furniture, construction, insulator to arms and backs, cushion thicking, dust covers, linings, stitch reinforcements, edge trim materials, bedding constructions, quilt backing, spring wrap, mattress pad components, mattress covers, window curtains, wall coverings, carpet backings, lampshades, mattress components, spring insulators, sealings, pillow ticking, and mattress ticking.

In yet another embodiment, the nonwoven cellulose fiber fabric may be used for manufacturing industrial products. This may involve electronics, floppy disc liners, cable insulation, abrasives, insulation tapes, conveyor belts, noise absorbent layers, air conditioning, battery separators, acid systems, anti-slip matting stain removers, food wraps, adhesive tape, sausage casing, cheese casing, artificial leather, oil recovery booms and socks, and papermaking felts.

Nonwoven cellulose fiber fabric according to exemplary embodiments of the invention is also appropriate for manufacturing products related to leisure and travel. Examples for such an application are sleeping bags, tents, luggage, handbags, shopping bags, airline headrests, CD-protection, pillowcases, and sandwich packaging.

Still another field of application of exemplary embodiment of the invention relates to school and office products. As examples, book covers, mailing envelopes, maps, signs and pennants, towels, and flags shall be mentioned.

### Brief description of the drawings

The invention will be described in more detail hereinafter with reference to examples of embodiment but to which the invention is not limited:
Figure 1 illustrates a device for manufacturing nonwoven cellulose fiber fabric which is directly formed from lyocell spinning solution being coagulated by a coagulation fluid according to an exemplary embodiment of the invention.
Figure 2 to Figure 4 show experimentally captured images of nonwoven cellulose fiber fabric according to an exemplary embodiment of the invention in which merging of individual fibers has been accomplished by a specific process control.
Figure 5 and Figure 6 show experimentally captured images of nonwoven cellulose fiber fabric according to an exemplary embodiment of the invention in which swelling of fibers has been accomplished, wherein Figure 5 shows the fiber fabric in a dry non-swollen state and Figure 6 shows the fiber fabric in a humid swollen state.
Figure 7 shows an experimentally captured image of nonwoven cellulose fiber fabric according to an exemplary embodiment of the invention in which formation of two superposed layers of fibers has been accomplished by a specific process implementing two serial bars of nozzles.
Figure 8 shows an experimentally captured image of a portion of a nonwoven cellulose fiber fabric according to an exemplary embodiment of the invention in which formation of a plurality of small primary or first pores is accomplished by a corresponding process control.
Figure 9 shows an experimentally captured image of another portion of the nonwoven cellulose fiber fabric according to Figure 8 in which formation of a plurality of large secondary or second pores is accomplished by a corresponding process control, more precisely by hydroentangling.
Figure 10 is a schematic illustration of a nonwoven cellulose fiber fabric according to an exemplary embodiment of the invention having different fabric portions with different plurality of pores having different sizes within different size ranges and being shown in a dry fabric state.
Figure 11 is a schematic illustration of the nonwoven cellulose fiber fabric according to Figure 10 and being shown in a wet fabric state.
Figure 12 is a schematic illustration of a portion of a nonwoven cellulose fiber fabric according to an exemplary embodiment of the invention showing changes of cavity dimensions when converting the fabric from a dry fiber state into a wet fiber state.
Figure 13 shows a schematic image of a nonwoven cellulose fiber fabric according to still another exemplary embodiment of the invention composed of two stacked and merged layers of interconnected fibers having different fiber thicknesses and differently sized pores.
Figure 14 illustrates a part of a device for manufacturing nonwoven cellulose fiber fabric composed of two stacked layers of endless cellulose fiber webs according to an exemplary embodiment of the invention.
Figure 15 shows a schematic image of a nonwoven cellulose fiber fabric according to still another exemplary embodiment of the invention composed of two stacked layers of interconnected fibers having different cavities defined between respective fibers and being filled with two different active agents being released sequentially rather than simultaneously.

### Detailed description of the drawings

The illustrations in the drawings are schematic. In different drawings similar or identical elements are provided with the same reference labels.

**Figure 1** illustrates a device 100 according to an exemplary embodiment of the invention for manufacturing nonwoven cellulose fiber fabric 102 which is directly formed from lyocell spinning solution 104. The latter is at least partly coagulated by a coagulation fluid 106 to be converted into partly-formed cellulose fibers 108. By the device 100, a lyocell solution blowing process according to an exemplary embodiment of the invention may be carried out. In the context of the present application, the term "lyocell solution-blowing process" may particularly encompass processes which can result in essentially endless filaments or fibers 108 of a discrete length or mixtures of endless filaments and fibers of discrete length being obtained. As further described below, nozzles each having an orifice 126 are provided through which cellulose solution or lyocell spinning solution 104 is ejected together with a gas stream or gas flow 146 for manufacturing the nonwoven cellulose fiber fabric 102 according to an exemplary embodiment of the invention.

As can be taken from Figure 1, wood pulp 110, other cellulose-based feedstock or the like may be supplied to a storage tank 114 via a metering unit 113. Water from a water container 112 is also supplied to the storage tank 114 via metering unit 113. Thus, the metering unit 113, under control of a control unit 140 described below in further detail, may define relative amounts of water and wood pulp 110 to be supplied to the storage tank 114. A solvent (such as N-methyl-morpholine, NMMO) accommodated in a solvent container 116 may be concentrated in a concentration unit 118 and may then be mixed with the mixture of water and wood pulp 110 or other cellulose-based feedstock with definable relative amounts in a mixing unit 119. Also the mixing unit 119 can be controlled by the control unit 140. Thereby, the water-wood pulp 110 medium is dissolved in the concentrated solvent in a dissolving unit 120 with adjustable relative amounts, thereby obtaining lyocell spinning solution 104. The aqueous lyocell spinning solution 104 can be a honey-viscous medium composed of (for instance 5 mass % to 15 mass %) cellulose comprising wood pulp 110 and (for instance 85 mass % to 95 mass %) solvent.

The lyocell spinning solution 104 is forwarded to a fiber formation unit 124 (which may be embodied as or which may comprise a number of spinning beams or jets 122). For instance, the number of orifices 126 of the jets 122 may be larger than 50, in particular larger than 100. In one embodiment, all orifices 126 of a fiber formation unit 124 (which may comprise a number of spinnerets of jets 122) of orifices 126 of the jets 122 may have the same size and/or shape. Alternatively, size and/or shape of different orifices 126 of one jet 122 and/or orifices 126 of different jets 122 (which may be arranged serially for forming a multilayer fabric) may be different.

When the lyocell spinning solution 104 passes through the orifices 126 of the jets 122, it is divided into a plurality of parallel strands of lyocell spinning solution 104. A vertically oriented gas flow, i.e. being oriented substantially parallel to spinning direction, forces the lyocell spinning solution 104 to transform into increasingly long and thin strands which can be adjusted by changing the process conditions under control of control unit 140. The gas flow may accelerate the lyocell spinning solution 104 along at least a part of its way from the orifices 126 to a fiber support unit 132.

While the lyocell spinning solution 104 moves through the jets 122 and further downward, the long and thin strands of the lyocell spinning solution 104 interact with non-solvent coagulation fluid 106. The coagulation fluid 106 is advantageously embodied as a vapor mist, for instance an aqueous mist. Process relevant properties of the coagulation fluid 106 are controlled by one or more coagulation units 128, providing the coagulation fluid 106 with adjustable properties. The coagulation units 128 are controlled, in turn, by control unit 140. Preferably, respective coagulation units 128 are provided between the individual nozzles or orifices 126 for individually adjusting properties of respective layers of fabric 102 being produced. Preferably, each jet 122 may have two assigned coagulation units 128, one from each side. The individual jets 122 can thus be provided with individual portions of lyocell spinning solution 104 which may also be adjusted to have different controllable properties of different layers of manufactured fabric 102.

When interacting with the coagulation fluid 106 (such as water), the solvent concentration of the lyocell spinning solution 104 is reduced, so that the cellulose of the former e.g. wood pulp 110 (or other feedstock) is at least partly coagulated as long and thin cellulose fibers 108 (which may still contain residual solvent and water).

During or after initial formation of the individual cellulose fibers 108 from the extruded lyocell spinning solution 104, the cellulose fibers 108 are deposited on fiber support unit 132, which is here embodied as a conveyor belt with a planar fiber accommodation surface. The cellulose fibers 108 form a nonwoven cellulose fiber fabric 102 (illustrated only schematically in Figure 1). The nonwoven cellulose fiber fabric 102 is composed of continuous and substantially endless filaments or fibers 108.

Although not shown in Figure 1, the solvent of the lyocell spinning solution 104 removed in coagulation by the coagulation unit 128 and in washing in a washing unit 180 can be at least partially recycled.

While being transported along the fiber support unit 132, the nonwoven cellulose fiber fabric 102 can be washed by washing unit 180 supplying wash liquor to remove residual solvent and may then be dried. It can be further processed by an optional but advantageous further processing unit 134. For instance, such a further processing may involve hydro-entanglement, needle punching, impregnation, steam treatment with a pressurized steam, calendering, etc.

The fiber support unit 132 may also transport the nonwoven cellulose fiber fabric 102 to a winder 136 on which the nonwoven cellulose fiber fabric 102 may be collected as a substantially endless sheet. The nonwoven cellulose fiber fabric 102 may then be shipped as roll-good to an entity manufacturing products such as wipes or textiles based on the nonwoven cellulose fiber fabric 102.

As indicated in Figure 1, the described process may be controlled by control unit 140 (such as a processor, part of a processor, or a plurality of processors). The control unit 140 is configured for controlling operation of the various units shown in Figure 1, in particular one or more of the metering unit 113, the mixing unit 119, the fiber formation unit 124, the coagulation unit(s) 128, the further processing unit 134, the dissolution unit 120, the washing unit 118, etc. Thus, the control unit 140 (for instance by executing computer executable program code, and/or by executing control commands defined by a user) may precisely and flexibly define the process parameters according to which the nonwoven cellulose fiber fabric 102 is manufactured. Design parameters in this context are air flow along the orifices 126, properties of the coagulation fluid 106, drive speed of the fiber support unit 132, composition, temperature and/or pressure of the lyocell spinning solution 104, etc. Additional design parameters which may be adjusted for adjusting the properties of the nonwoven cellulose fiber fabric 102 are number and/or mutual distance and/or geometric arrangement of the orifices 126, chemical composition and degree of concentration of the lyocell spinning solution 104, etc. Thereby, the properties of the nonwoven cellulose fiber fabric 102 may be properly adjusted, as described below. Such adjustable properties (see below detailed description) may involve one or more of the following properties: diameter and/or diameter distribution of the fibers 108, amount and/or regions of merging between fibers 108, a purity level of the fibers 108, properties of a multilayer fabric 102, optical properties of the fabric 102, fluid retention and/or fluid release properties of the fabric 102, mechanical stability of the fabric 102, smoothness of a surface of the fabric 102, cross-sectional shape of the fibers 108, etc.

Although not shown, each spinning jet 122 may comprise a polymer solution inlet via which the lyocell spinning solution 104 is supplied to the jet 122. Via an air inlet, a gas flow 146 can be applied to the lyocell spinning solution 104. Starting from an interaction chamber in an interior of the jet 122 and delimited by a jet casing, the lyocell spinning solution 104 moves or is accelerated (by the gas flow 146 pulling the lyocell spinning solution 104 downwardly) downwardly through a respective orifice 126 and is laterally narrowed under the influence of the gas flow 146 so that continuously tapering cellulose filaments or cellulose fibers 108 are formed when the lyocell spinning solution 104 moves downwardly together with the gas flow 146 in the environment of the coagulation fluid 106.

Thus, processes involved in the manufacturing method described by reference to Figure 1 may include that the lyocell spinning solution 104, which may also be denoted as cellulose solution is shaped to form liquid strands or latent filaments, which are drawn by the gas flow 146 and significantly decreased in diameter and increased in length. Partial coagulation of latent filaments or fibers 108 (or preforms thereof) by coagulation fluid 106 prior to or during web formation on the fiber support unit 132 may also be involved. The filaments or fibers 108 are formed into web like fabric 102, washed, dried and may be further processed (see further processing unit 134), as required. The filaments or fibers 108 may for instance be collected, for example on a rotating drum or belt, whereby a web is formed.

As a result of the described manufacturing process and in particular the choice of solvent used, the fibers 108 have a copper content of less than 5 ppm and have a nickel content of less than 2 ppm. This advantageously improves purity of the fabric 102.

The lyocell solution blown web (i.e. the nonwoven cellulose fiber fabric 102) according to exemplary embodiments of the invention preferably exhibits one or more of the following properties:
(i) The dry weight of the web is from 5 to 300 g/m², preferably 10-80 g/m²
(ii) The thickness of the web according to the standard WSP120.6 respectively DIN29073 (in particular in the latest version as in force at the priority date of the present patent application) is from 0.05 to 10.0 mm, preferably 0.1 to 2.5 mm
(iii) The specific tenacity of the web in MD according to EN29073-3, respectively IS09073-3 (in particular in the latest version as in force at the priority date of the present patent application) ranges from 0.1 to 3.0 Nm²/g, preferably from 0.4 to 2.3 Nm²/g
(iv) The average elongation of the web according to EN29073-3, respectively IS09073-3 (in particular in the latest version as in force at the priority date of the present patent application) ranges from 0.5 to 100%, preferably from 4 to 50%.
(v) The MD/CD tenacity ratio of the web is from 1 to 12
(vi) The water retention of the web according to DIN 53814 (in particular in the latest version as in force at the priority date of the present patent application) is from 1 to 250%, preferably 30 to 150%
(vii) The water holding capacity of the web according to DIN 53923 (in particular in the latest version as in force at the priority date of the present patent application) ranges from 90 to 2000%, preferably 400 to 1100%.
(viii) Metal residue levels of copper content of less than 5 ppm and nickel content of less than 2 ppm, according to the standards EN 15587-2 for the substrate decomposition and EN 17294-2 for the ICP-MS analysis (in particular in the latest version as in force at the priority date of the present patent application)

Most preferably, the lyocell solution-blown web exhibits all of said properties (i) to (viii) mentioned above.

As described, the process to produce the nonwoven cellulose fiber fabric 102 preferably comprises:
(a) Extruding a solution comprising cellulose dissolved in NMMO (see reference numeral 104) through the orifices 126 of at least one jet 122, thereby forming filaments of lyocell spinning solution 104
(b) Stretching said filaments of lyocell spinning solution 104 by a gaseous stream (see reference numeral 146)
(c) Contacting said filaments with a vapor mist (see reference numeral 106), preferably containing water, thereby at least partly precipitating said fibers 108. Consequently, the filaments or fibers 108 are at least partly precipitated before forming web or nonwoven cellulose fiber fabric 102.
(d) Collecting and precipitating said filaments or fibers 108 in order to form a web or nonwoven cellulose fiber fabric 102
(e) Removing solvent in wash line (see washing unit 180)
(f) Optionally bonding via hydro-entanglement, needle punching, etc. (see further processing unit 134)
(g) Drying and roll collection

Constituents of the nonwoven cellulose fiber fabric 102 may be bonded by merging, intermingling, hydrogen bonding, physical bonding such as hydroentanglement or needle punching, and/or chemical bonding.

In order to be further processed, the nonwoven cellulose fiber fabric 102 may be combined with one or more layers of the same and/or other materials, such as (not shown) layers of synthetic polymers, cellulosic fluff pulp, nonwoven webs of cellulose or synthetic polymer fibers, bicomponent fibers, webs of cellulose pulp, such as airlaid or wetlaid pulp, webs or fabrics of high tenacity fibers, hydrophobic materials, high performance fibers (such as temperature resistant materials or flame retardant materials), layers imparting changed mechanical properties to the final products (such as Polypropylene or Polyester layers), biodegradable materials (e.g. films, fibers or webs from Polylactic acid), and/or high bulk materials.

It is also possible to combine several distinguishable layers of nonwoven cellulose fiber fabric 102, see for instance Figure 7.

The nonwoven cellulose fiber fabric 102 may essentially consist of cellulose alone. Alternatively, the nonwoven cellulose fiber fabric 102 may comprise a mixture of cellulose and one or more other fiber materials. The nonwoven cellulose fiber fabric 102, furthermore, may comprise a bicomponent fiber material. The fiber material in the nonwoven cellulose fiber fabric 102 may at least partly comprise a modifying substance. The modifying substance may be selected from, for example, the group consisting of a polymeric resin, an inorganic resin, inorganic pigments, antibacterial products, nanoparticles, lotions, fire-retardant products, absorbency-improving additives, such as superabsorbent resins, ion-exchange resins, carbon compounds such as active carbon, graphite, carbon for electrical conductivity, X-ray contrast substances, luminescent pigments, and dye stuffs.

Concluding, the cellulose nonwoven web or nonwoven cellulose fiber fabric 102 manufactured directly from the lyocell spinning solution 104 allows access to value added web performance which is not possible via staple fiber route. This includes the possibility to form uniform lightweight webs, to manufacture microfiber products, and to manufacture continuous filaments or fibers 108 forming a web. Moreover, compared to webs from staple fibers, several manufacturing procedures are no longer required. Moreover, nonwoven cellulose fiber fabric 102 according to exemplary embodiments of the invention is biodegradable and manufactured from sustainably sourced raw material (i.e. wood pulp 110 or the like). Furthermore, it has advantages in terms of purity and absorbency. Beyond this, it has an adjustable mechanical strength, stiffness and softness. Furthermore, nonwoven cellulose fiber fabric 102 according to exemplary embodiments of the invention may be manufactured with low weight per area (for instance 10 to 30 g/m²). Very fine filaments down to a diameter of not more than 5 µm, in particular not more than 3 µm, can be manufactured with this technology. Furthermore, nonwoven cellulose fiber fabric 102 according to an exemplary embodiment of the invention may be formed with a wide range of web aesthetics, for instance in a flat crispy film-like way, in a paper-like way, or in a soft flexible textile-like way. By adapting the process parameters of the described process, it is furthermore possible to precisely adjust stiffness and mechanical rigidity or flexibility and softness of the nonwoven cellulose fiber fabric 102. This can be adjusted for instance by adjusting a number of merging positions, the number of layers, or by after-treatment (such as needle punch, hydro-entanglement and/or calendering). It is in particular possible to manufacture the nonwoven cellulose fiber fabric 102 with a relatively low basis weight of down to 10 g/m² or lower, to obtain filaments or fibers 108 with a very small diameter (for instance of down to 3 to 5 µm, or less), etc.

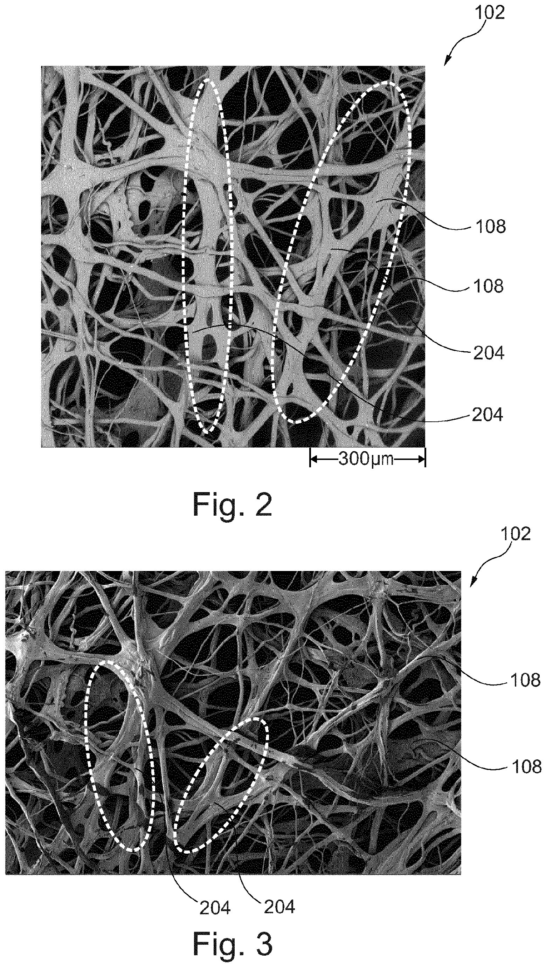

**Figure 2, Figure 3** and **Figure 4** show experimentally captured images of nonwoven cellulose fiber fabric 102 according to an exemplary embodiment of the invention in which merging of individual fibers 108 has been accomplished by a corresponding process control. The oval markers in Figure 2 to Figure 4 show such merging regions where multiple fibers 108 are integrally connected to one another. At such merging points, two or more fibers 108 may be interconnected to form an integral structure.

**Figure 5** and **Figure 6** show experimentally captured images of nonwoven cellulose fiber fabric 102 according to an exemplary embodiment of the invention in which swelling of fibers 108 has been accomplished, wherein Figure 5 shows the fiber fabric 102 in a dry non-swollen state and Figure 6 shows the fiber fabric 102 in a humid swollen state. The pore diameters can be measured in both states of Figure 5 and Figure 6 and can be compared to one another. When calculating an average value of 30 measurements, a decrease of the pore size by swelling of the fibers 108 in an aqueous medium up to 47% of their initial diameter could be determined.

**Figure 7** shows an experimentally captured image of nonwoven cellulose fiber fabric 102 according to an exemplary embodiment of the invention in which formation of two superposed layers 200, 202 of fibers 108 has been accomplished by a corresponding process design, i.e. a serial arrangement of multiple spinnerets. The two separate, but connected layers 200, 202 are indicated by a horizontal line in Figure 7. For instance, an n-layer fabric 102 (n≥2) can be manufactured by serially arranging n spinnerets or jets 122 along the machine direction.

Specific exemplary embodiments of the invention will be described in the following in more detail:
**Figure 8** shows an experimentally captured image of a portion of a nonwoven cellulose fiber fabric 102 according to an exemplary embodiment of the invention in which formation of a plurality of smaller first pores 260 is accomplished by a corresponding process control of the manufacturing method. **Figure 9** shows an experimentally captured image of another portion of the nonwoven cellulose fiber fabric 102 according to Figure 8 in which formation of a plurality of larger second pores 264 is accomplished by a corresponding process control of the manufacturing method. In order to manufacture the fabric 102 shown in Figure 8 and Figure 9, it is possible to configure the control unit 140 of Figure 1 for adjusting process parameters so that the fabric 102 is formed with smaller first pores 260 (compare Figure 8) and with larger second pores 264 (compare Figure 9). For instance, the plurality of second pores 264 may be formed by hydro-entanglement selectively of the corresponding fabric portion or of the entire fabric. Thereby, the relatively large voids forming the second pores 264 may be formed in the fabric 102 by means of high pressure jets of water. Thus, the second pores 264 may be formed by displacing fibers 108 to thereby form voids between fiber bundles. Hence, the second pores 264 may be manufactured by patterning a homogeneous fiber network to thereby obtain a patterned fiber network. In contrast to this, the plurality of first pores 260 may be formed as a high density web of fibers 108. In other words, the first pores 260 are defined as fiber-fiber or inter-fiber distances. Thus, the first pores 260 may be manufactured by simply using a homogeneous fiber network.

As a result of the manufacture of endless fibers 108 by the device 100 and the method described referring to Figure 1, it is possible to obtain the fabric shown in Figure 8 and Figure 9 with a very small amount of fiber ends per volume of for instance less than 5,000 ends/cm³. A further consequence of the described manufacturing procedure is that the fibers 108 have a copper content of less than 5 ppm and have a nickel content of less than 2 ppm. This depletion of undesired heavy metals in the fabric 102 is the result of a corresponding adjustment of the process parameters which neither involves heavy metal sources (for instance does not use copper salt solution) in the manufacturing procedure nor brings the used operating fluids (such as lyocell spinning solution 104, coagulation fluid 106, gas flow 146, etc.) or the readily manufactured fibers 108 into contact with heavy metal sources.

The fabric 102 according to Figure 8 and Figure 9 may be configured so that a wicking speed is at least 0. 25 g water/g fabric/s. Consequently, medium may quickly enter or exit the first pores 260, and medium can be injected rapidly in the second pores 264 and/or released therefrom.

Figure 8 shows a substantially homogeneous fiber distribution as obtained from extruded lyocell spinning solution 104 without the need of further processing. In contrast to this, Figure 9 shows an inhomogeneous fiber distribution as obtained from extruded lyocell spinning solution 104 followed by hydro-entanglement which creates the second pores 264. When the secondary pores 264 are formed by hydroentanglement or needling, the secondary pores 264 may be arranged in accordance with a defined spatial order, for instance in a matrix pattern. However, in other embodiments, the arrangement of the secondary pores 264 can also be randomly distributed. Also the primary pores 260 may be arranged with spatial order or randomly distributed.

As can be taken from a comparison of Figure 8 with Figure 9, the plurality of first pores 260 are provided over the entire fabric 102, whereas the plurality of second pores 264 are provided only over a hydroentangled subsection of the fabric 102. In particular, the fabric regions between the second pores 264 shown in Figure 9 may be also provided with the first pores 260 shown in Figure 8. This can be obtained by a selective formation of the second pores 264 by hydroentanglement, whereas the first pores 260 are generated automatically as fiber-fiber gaps during formation of the fabric 102.

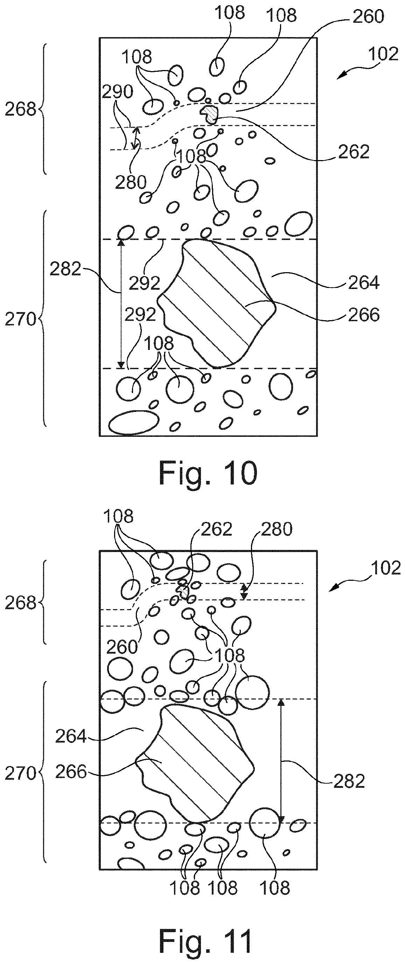

**Figure 10** **is** a schematic illustration of a nonwoven cellulose fiber fabric 102 according to an exemplary embodiment of the invention having different fabric portions 268, 270 with different plurality of pores 260, 264 having different sizes 280, 282 within different size ranges and being shown in a dry fabric state (i.e. without water within the fibers 108). **Figure 11** is a schematic illustration of the nonwoven cellulose fiber fabric 102 according to Figure 10 and being shown in a wet fabric state (i.e. in a state in which the fibers 108 have soaked water to thereby become swollen and/or spatially displaced). The nonwoven cellulose fiber fabric 102 according to Figure 10 and Figure 11 is directly manufactured from lyocell spinning solution 104 and comprises a network of substantially endless fibers 108. Figure 10 and Figure 11 describe a fiber swelling based particle retaining and release mechanism.

The fabric 102 comprises a first fabric portion 268 having a plurality of first pores 260 delimited between a first plurality of the fibers 108. The first pores 260 (only one is shown in Figure 10) correspond to a first minimum required path (see reference numeral 290) for a first particle 262 accommodated within one of the first pores 260 to get out of the fabric 102. In the dry state of the fabric 102 shown in Figure 10, the dimension of the first particle 262 is so that it is capable of moving along the path according to reference numeral 290 to get into the fabric 102 or out of the fabric 102. In other words, the first pores 260 have sizes 280 within a first size range and are configured for retaining or releasing the first particle(s) 262.

Apart from this, the fabric 102 comprises a plurality of second pores 264. The second pores 264 (only one is shown in Figure 10) correspond to a second minimum required path (see reference numeral 292) for a larger second particle 266 accommodated within one of the second pores 264 to get out of the fabric 102. In the dry state of the fabric 102 shown in Figure 10, the dimension of the second particle 266 is so that it is capable of moving along the path according to reference numeral 292 to get into the fabric 102 or out of the fabric 102. In other words, the second pores 264 have sizes 282 within a second size range and are configured for retaining or releasing the second particle(s) 266. The first size range encompasses sizes 280 being smaller than sizes 282 encompassed by the second size range. The first size range and the second size range may be different size ranges, in particular may have no size 280, 282 in common.

The endless fibers 108 comprising microfibrillar cellulose, are configured so that the plurality of first pores 260 and the plurality of second pores 264 modify the respective size range by moisture induced fiber swelling. According to Figure 11, moisture has been added to the fabric 102 which results in a modification of the network of fibers 108 so that fibers 108 now extend into the minimum required paths (see reference numerals 290, 292) and prevent the respective particles 262, 266 from leaving the fabric 102. Thus, the fabric 102 can be converted from a state shown in Figure 10 in which the particles 262, 266 can freely enter the fabric 102 or leave the fabric 102 and are therefore in a released condition, into another state shown in Figure 11 in which the particles 262, 266 are retained (or clamped or locked) within the fabric 102. If the fabric 102 shown in Figure 11 is again dried (for instance by a temperature triggered evaporation of the moisture out of the fabric 102 ), the fibers 108 will again shrink and will therefore return into the release state shown in Figure 10.

Concluding, in the dry state according to Figure 10, the first particles 262 are enabled to enter into or leave the first pores 260, and the second particles 266 are enabled to enter into or leave the second pores 264. In contrast to this, in the wet state according to Figure 11, both the first particles 262 and the second particles 266 are disabled to enter into or leave the first pores 260 and the second pores 264, respectively. Adding moisture to the fabric 102 can therefore be carried out for adjusting a decrease of the size ranges by moisture-based swelling of the fibers 108. Removing moisture from the fabric 102 will increase the size ranges by moisture-based shrinkage of the fibers 108. Therefore, it is possible to use the nonwoven cellulose fiber fabric 102 according to an exemplary embodiment of the invention for adjusting a humidity state of the fibers 108 for modifying the respective size ranges of the plurality of first pores 260 and the plurality of second pores 264, to thereby adjust the particle retaining or releasing properties of the fabric 102.

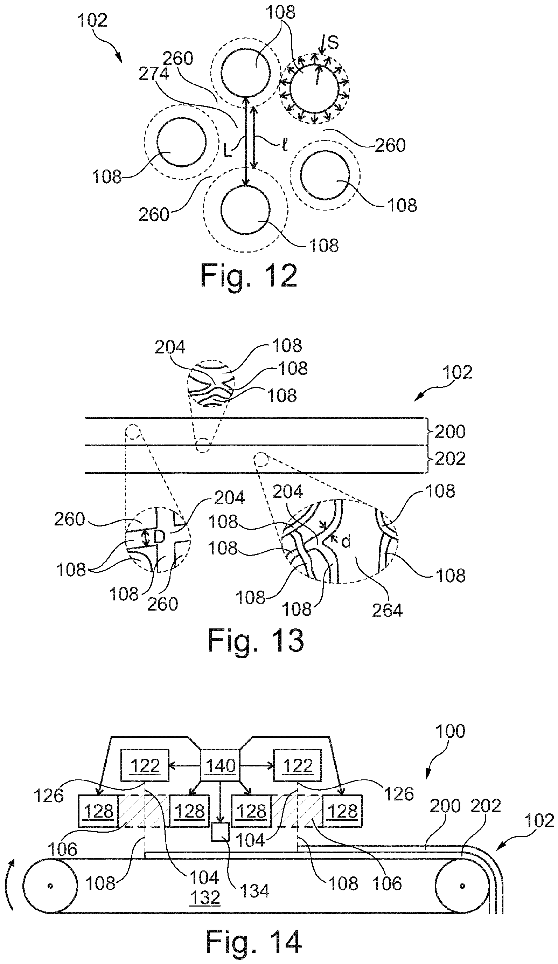

**Figure 12** is a schematic illustration of a portion of a nonwoven cellulose fiber fabric 102 according to an exemplary embodiment of the invention showing changes of cavity dimensions L,I of a cavity 274 by converting the fabric 102 from a dry fiber state into a wet fiber state.

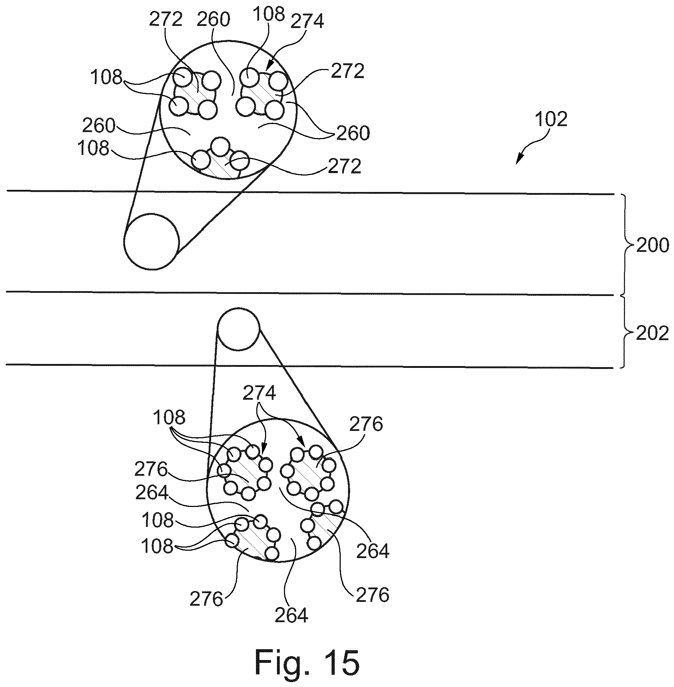

As can be taken from Figure 12, the fabric 102 is configured so that cavities 274 (one thereof is shown in Figure 12) are defined or delimited between fibers 108. In the shown embodiment, five fibers 108 surround and thereby delimit a cavity 274 in which a particle 262, 266 or an active agent 272, 276 (compare Figure 15) may be accommodated. In order to transform the fabric 102 between a medium retaining condition (corresponding to swollen fibers 108 and therefore closed cavities 274) and a medium releasing condition (corresponding to shrunk fibers 108 and therefore open cavities 274), it is possible to trigger the fibers 108 to change diameter (see arrows in Figure 12 indicating an increase of a fiber diameter by "s" in the event of moisture induced swelling of the shown fiber 108). As indicated in Figure 12, the cavities or pores may undergo a diameter decrease of for instance 20% from a dry state to a wet state of the fabric 102. Correspondingly, the cavity 274 (delimited between fibers 108) may undergo a diameter decrease from "L" to "I" from the dry state to the wet state of the fabric 102.

A dry shrunk state of the fibers 108 is indicated by solid lines in Figure 12. Correspondingly, a wet swollen state of the fibers 108 is indicated by dashed lines in Figure 12. By soaking moisture, the radius of the fibers 108 increases by a distance s. Since the cavity 274 is delimited by the fibers 108, this transition from the shrunk into the swollen state of the fibers 108 reduces the diameter of the cavity 274 from L to I.

**Figure 13** shows a schematic cross sectional view of a nonwoven cellulose fiber fabric 102 according to an exemplary embodiment of the invention composed of two stacked and merged layers 200, 202 of interconnected fibers 108 having different fiber thicknesses d and D>d (see the lower two details of Figure 13). More specifically, different ones of the fibers 108 being located in the different layers 200, 202 differ concerning an averaged fiber diameter (i.e. averaged over the fibers 108 of the respective layer 200, 202). Fibers 108 of the respective layers 200, 202 are also merged at merging positions 204, compare the lower two details of Figure 13. A further detail of the interface between the layers 200, 202 is shown as well, where a merging point 204 is visible which integrally couples fibers 108 of both layers 200, 202 at the interface for increasing stability of the fabric 102 at the interface (see the upper detail of Figure 13). Additionally, different ones of the fibers 108 being located in the different layers 200, 202 are integrally connected at at least one respective merging position 204.

Merging properties may be adjusted to obtain desired properties, also in terms of retaining or releasing a medium (see reference numerals 262, 266, 272, 276) in or from fabric 102. For instance, a number of merging points 204 per volume of fabric 102 may be adjusted separately within the respective one of the layers 200, 202 and/or between the layers 200, 202. This can be done by adjusting the coagulation properties (in particular coagulation of filaments of lyocell spinning solution 104 upstream of the fiber accommodation surface of the fiber support unit 132, coagulation of filaments of lyocell spinning solution 104 after lay down of the filaments on the fiber accommodation surface of the fiber support unit 132, etc.). The merging between the different layers 200, 202 may be adjusted so that pulling on the layers 200, 202 in opposite directions results in a separation of the fabric 102 at an interface between the different layers 200, 202. In other words, merging-based adhesion between the different layers 200, 202 may be adjusted to be smaller than merging based adhesion within a respective one of the different layers 200, 202.

The fibers 108 located in the different layers 200, 202 and being formed with different average diameter and different merging properties may be provided with different functionalities. Such different functionalities may be supported by the different average diameters, but may also be further promoted by a respective coating or the like. Such different functionalities may for instance be a different behavior in terms of wicking speed, anisotropic behavior, different oil absorbing capability, different water absorbing capability, different cleanability, and/or different roughness.

The multilayer nonwoven cellulose fiber fabric 102 according to Figure 13 can be directly manufactured from lyocell spinning solution 104 using the device 100 and corresponding manufacturing method described below referring to Figure 14. Advantageously, the partial heavy metal contaminations of the fibers 108 of the fabric 102 according to Figure 13 are not more than 10 ppm for each individual chemical heavy metal element (i.e. not more than 10 ppm for iron, not more than 10 ppm for zinc, not more than 10 ppm for cadmium, etc.). Beyond this, an overall or entire heavy metals content of fabric 102 summed up for all heavy metal chemical elements together (i.e. in particular for Cr, Mn, Fe, Co, Ni, Cu, Zn, Zr, Mo, Cd, Sn, W, Pb, Bi) is not more than 30 ppm. Apart from this, the fibers 108 have a copper content of less than 5 ppm and have a nickel content of less than 2 ppm. This as a consequence of the operating fluids (in particular lyocell spinning solution 104, coagulation fluid 106, washing liquor, gas flow 146, etc.) which are used during the manufacturing process and which may be substantially free of heavy metal sources such as copper salt. As a result of this design of the manufacturing process, the fibers 108 may be of high quality and may substantially consist of pure microfibrillar cellulose. The absence of any mentionable heavy metal impurities in the manufacturing process prevents highly undesired decomposition of involved media (in particular of the lyocell spinning solution 104) and therefore allows to obtain highly reproducible and highly pure cellulose fabric 102.

As can be taken from Figure 13, the various fibers 108 differ concerning fiber diameter so that a ratio between the 10% thinnest fibers 108 and the 10% thickest fibers 108 may be more than 0.05. For example, in one embodiment at least 97 mass percent of the fibers 108 have an average fiber diameter in a range between 3 µm and 15 µm.

As can be taken from Figure 13 as well, the dimension of first pores 260 in layer 200 is smaller than the dimension of second pores 264 in layer 202. For instance, the first pores 260 are fiber-fiber gaps, whereas the second pores 264 are significantly larger and may for instance be formed by hydro-entangling.

**Figure 14** illustrates a part of a device 100 for manufacturing nonwoven cellulose fiber fabric 102 composed of two stacked layers 200, 202 of endless cellulose fibers 108 according to an exemplary embodiment of the invention. A difference between the device 100 shown in Figure 14 and the device 100 shown in Figure 1 is that the device 100 according to Figure 14 comprises two serially aligned jets 122 and respectively assigned coagulation units 128, as described above. In view of the movable fiber accommodation surface of the conveyor belt-type fiber support unit 132, the upstream jet 122 on the left-hand side of Figure 14 produces layer 202. Layer 200 is produced by the jet unit 122 (see right hand side of Figure 14) and is attached to an upper main surface of the previously formed layer 202 so that a double layer 200, 202 of fabric 102 is obtained.

According to Figure 14, the control unit 140 (controlling the jets 122 and the coagulation units 128) is configured for adjusting process parameters so that the fibers 108 of the different layers 200, 202 differ concerning fiber diameter by more than 50% in relation to a smallest diameter (see for example Figure 13). Adjusting the fiber diameters of the fibers 108 of the layers 200, 202 by the control unit 140 may comprise adjusting an amount of coagulation fluid 106 interacting with the lyocell spinning solution 104. Additionally, the embodiment of Figure 14 adjusts the process parameters for adjusting fiber diameter by serially arranging multiple jets 122 with orifices 126 (optionally with different properties) along the movable fiber support unit 132. For instance, such different properties may be different orifice 126 diameters, different speed of gas flow 146, different amounts of gas flow 146, and/or different gas flow 146 pressure.

Still referring to the embodiment illustrated in Figure 14, one or more further nozzle bars or jets 122 may be provided and may be arranged serially along a transport direction of fiber support unit 132. The multiple jets 122 may be arranged so that further layer 200 of fibers 108 may be deposited on top of the previously formed layer 202, preferably before the coagulation or curing process of the fibers 108 of the layer 202 and/or of the layer 200 is fully completed, which may trigger merging. When properly adjusting the process parameters, this may have advantageous effects in terms of the properties of a multilayer fabric 102:
The device 100 according to Figure 14, which is configured for the manufacture of multilayer fabric 102, implements a high number of process parameters which can be used for designing shape and/or diameter or diameter distribution of the fibers 108 and of the pores 260, 264 as well as of fiber layers 200, 202. This is the result of the serial arrangement of jets 122, each of which being operable with individually adjustable process parameters.

As can be taken from Figure 14 as well, a further processing unit 134 (controlled by control unit 140) is arranged downstream of the first jet 122 forming layer 202, but upstream of the second jet 122 forming layer 200. Consequently, the further processing unit 134 will only further process layer 202, not layer 200. In the shown embodiment, the further processing unit 134 may for instance be a hydro-entangling unit configured for hydro-entangling selectively layer 202, but not layer 200. As a result, layer 202 may be provided with the larger second pores 264 generated as a result of hydro-entanglement, whereas layer 200 may be formed with the smaller first pores 260 obtained as fiber-fiber distances without further processing layer 200.

However, in yet another exemplary embodiment, it is possible that the further processing unit 134 is arranged downstream of a washing unit 180 (compare Figure 1). In such an embodiment, the entire fabric 102 may be provided with the secondary or second pores 264.

**Figure 15** shows a schematic image of a nonwoven cellulose fiber fabric 102 according to still another exemplary embodiment of the invention composed of two stacked layers 200, 202 of interconnected fibers 108 having different cavities 274 defined as hollow spaces between respective fibers 108 and being filled with two different active agents 272, 276. The speed of loading water into the fibers 108 may be controlled so that the active agents 272, 276 are released sequentially rather than simultaneously. With the shown fabric 102, a control release of active agents 272, 276 may be obtained.

The nonwoven cellulose fiber fabric 102 according to Figure 15 comprises two interconnected layers 200, 202 forming a network of substantially endless fibers 108. Both layers 200, 202 comprise pores, compare reference numerals 260, 264, delimited between fibers 108 or fiber groups. The respective pores 260, 264 are in fluid communication with cavities 274. The cavities 274 are volumes delimited by the fibers 108 as well and defining an accommodation space for a respective active agent 272, 276. More specifically, a first active agent 272 (such as a first pharmaceutical agent) is accommodated in cavities 274 in fluid communication with the first pores 260. Correspondingly, a second active agent 276 (such as a second pharmaceutical agent) is accommodated in cavities 274 in fluid communication with the second pores 264. For instance, for obtaining an optimum pharmaceutical impact of a medication in form of the active agent filled fabric 102, it may be desired that, for instance in a body of a human patient, the first active agent 272 shall be released first, and only thereafter the second active agent 276 shall be released. In order to achieve this, the different properties of the fiber networks in the different layers 200, 202 (in terms of fiber diameter, pore sizes, merging positions 204, etc.) may be taken into account. More precisely, in response to a change of one or more conditions to which the fabric 102 is subjected, the different layers 200, 202 may be impacted differently. Even more specifically, the fibers 108 in layer 200 have smaller first pores 260, have large diameters of the fibers 108 and have cavities 274 delimited by only four fibers 108. In contrast to this, the fibers 108 and layer 202 have larger second pores 264, have smaller diameters of the fibers 108 and have cavities 274 delimited by a larger number of fibers 108. This has an impact on the response of the respective layer 200, 202 to a change of one or more conditions in terms of active agent release properties.

For instance, it is possible to adjust a humidity state of the fibers 108 for triggering swelling or shrinkage of the fibers 108 in the respective layers 200, 202 to thereby control release of the respective active agent 272, 276 out of the cavities 274 via pores 260, 264. In particular, it may be advantageous to adjust the humidity state of the fibers 108 for triggering fiber swelling or shrinkage to thereby control release of the second active agent 276 out of the cavities 274 in fluid communication with the second pores 264 only after having completed the release of the first active agent 272 out of the cavities 274 in fluid communication with the first pores 260.

It is also possible to adjust another condition than the humidity state (for instance a mechanical tension of the fiber network, a temperature related parameter, etc.) of the fibers 108 are/or of the fabric 102 for triggering release of the first active agent 272 and subsequently of the second active agent 276.

By taking this measure, a product may be manufactured based on the fabric 102 filled with the two or more types of active agents 272, 276, in which it is precisely predictable that release of the second active agent 276 does not start before release of the first active agent 272 has been completed.

According to exemplary embodiments, a swelling characteristics of fibers 108 of a nonwoven cellulose fiber fabric 102 may be functionalized, i.e. may be used for precisely defining the fabric properties in terms of entering of a medium into the fabric 102, removal of a medium out of the fabric 102 and/or retaining and release properties of medium within the fabric 102. By controlling process parameters of manufacturing such a fabric 102, moisture-dependent swelling and shrinking behavior of the fibers 108 of the fabric 102 can be adjusted. More specifically, also a liquid spreading velocity (for example wicking speed) of the resulting product can be influenced by such a process control. In an embodiment, the swelling (or moisture expansion) capability of endless cellulose fibers 108 is used and is controlled for mechanically fixing particles 262, 266 or an active agent 272, 276 within the fabric 102. The manufacturing method may for instance be controlled in terms of adjusting fiber swelling properties by correspondingly adjusting process parameters such as diameter of the fibers 102, diameter distribution of the fibers 102, adjusting of merging positions 204 between fibers 108, adjusting crystallinity of the fibers 108, adjusting alpha cell content, etc. By adjusting an anisotropic alignment of pores 260, 264 between fibers 108, it is also possible to adjust anisotropic swelling behavior and/or anisotropic wicking speed of the fabric 102. Moreover, it is possible to add additives to one or more operating fluids (such as lyocell spinning solution 104, coagulation fluid 106, gas flow 146, washing liquor, etc.) during manufacture of the fibers 108 to thereby characteristically influence the swelling behavior or shrinkage behavior of the fibers 108 in the presence or absence of moisture. In particular, the fabric 102 may introduce and discharge particles 262, 266, and may have a selectivity in terms of particle size.

According to an embodiment, a nonwoven cellulose fiber fabric 102 is provided in order to ensure that a humidity induced swelling of fibers 108 may occur undisturbed over the entire volume of the fabric 102. Advantageously, the fibers 108 may be merged prior to completion of fiber formation, i.e. prior to completed coagulation or precipitation of the fiber 108. With a corresponding process control of the manufacturing method, it is possible to obtain a fiber network having primary pores 260 and (in particular partially open) cavities 274. Cellulose is properly wettable (descriptively speaking, its contacting angle may be significantly below 90°). As a result of the wettable surface of the fibers 108, a strong capillary action can be obtained. As a consequence, present moisture may spread and distribute quickly and may therefore trigger a systematic swelling of the fibers 108 with a predictable swelling speed. In particular, a ratio between swelling behavior and areal moisture spreading may be precisely controlled via process parameters of the manufacturing method. Such a capillary suction effect can also be used for carrying along particles, which can then be trapped in the fabric 102 after swelling.

The inverse of the swelling procedure, i.e. a shrinking procedure, functions in a reversible way: due to the high degree of merging between the fibers 108 defining cavities 274, a uniform moisture equilibration characteristic can be obtained, which in turn has the consequence of a uniform shrinking behavior of the fibers 108 within the fabric 102.

By modifying the moisture content of the nonwoven cellulose fiber fabric 102 according to an exemplary embodiment of the invention, a mechanical modification of the fiber geometry may be achieved. For instance, such a modification may comprise a modification of opening diameters. Such a modification may be used for a precisely controlled introduction of particles 262, 266 into the fabric 102 or removal of such particles 262, 266 from the fabric 102 towards an environment. In particular, it is possible to control swelling velocity of the fibers 108, thereby controlling opening times and closing times of the cavity 274.

It is also for instance possible to vary the crystallinity of the fibers 108 for controlling the swelling properties of the fibers 108. For instance, stretching of the strands of lyocell spinning solution 104 supported by gas flow 146 may be an appropriate process parameter for adjusting moisture controlled swelling of the fibers 108.

The advantageous implementation of endless fibers 108 in the fabric 102 provides for a liquid distribution along the entire length of the fiber 108 and not only within a short fiber section. The low number of fiber ends per volume obtainable with the manufacturing method described referring to Figure 1 and Figure 14 is advantageous in this respect.

Another process parameter which can be adjusted for influencing the pore properties and swelling capability of the fabric 102 is a twisting of at least part of the fibers 108 during the manufacturing procedure. The medium retaining properties of the fabric 102 can be further improved by taking this measure, because a multidimensional tension distribution is generated with twisted fibers 108, in particular upon swelling. Twisted fibers 108 may also provide an enhanced capillary effect.

A fabric 102 according to an exemplary embodiment of the invention may be used for manufacturing a wipe, in particular an industrial wipe. For a professional wipe, it may be advantageous that a diameter range of particles 262, 266 which can be absorbed or collected by a wipe is known in advance. By controlling the pore sizes of the fabric 102 during manufacture (for instance by hydro-entangling), the mentioned diameter range of absorbable or collectable particles 262, 266 may be predicted as well. A corresponding wipe may be a multiple use wipe. A wet wipe having absorbed or collected particles 262, 266 during a cleaning procedure may be subsequently dried which may result in a shrinking of the fibers 108. With a corresponding configuration of the fabric 102, shrinking of the fibers 108 may even trigger an increase of the pore sizes and a reduction of the adhesive forces so that the absorbed or collected particles 262, 266 may be simply removed from the wipe. The particles 262, 266 may even automatically fall out of the fabric 102. Alternatively, removal of the particles 262, 266 from the fabric 102 may be promoted by rocking or shaking the fabric 102. Highly advantageously, the heavy metal contamination of such a fabric 102 can be very low as a result of the formation of the fabric 102 on the basis of a lyocell spinning solution 104. A similar property can also be obtained by carrying out a suspension spinning method, to obtain microfibrillar or nanofibrillar cellulose.

A fabric 102 according to an exemplary embodiment of the invention may be used for a wetfloor or for a mop for a wet cleaning of surfaces. Such a product, activated by the supply of water, may allow to obtain an increased adhesion for dust particles 262, 266 and their retention within the fabric 102. On the one hand, a slow swelling of the fiber 102 made of cellulose can ensure that the adhesion remains for a sufficiently long time period. On the other hand, a sufficiently quick liquid spreading can be achieved in view of the substantially endless fibers 108 of the fabric 102. Controlled merging and fiber diameter variations can be adjusted by correspondingly adjusting process parameters of the manufacturing method.

A fabric 102 according to an exemplary embodiment of the invention may be used for a medical product with controlled delivery of active substances. For instance, the fabric 102 may form the basis of a band-aid or a medical bandage which may be filled with one, two or more active agents 272, 276. When covering a wound, such a medical product may rapidly activate release of one or more active agents 272, 276 (for instance a disinfectant) triggered by a contact of (even a very small surface area of) the fabric 102 with a body fluid. By the controllable swelling speed the delay until release of a respective active agent 272, 276 can be controlled. Also for the described medical application, it is highly advantageous that the fabric 102 can be manufactured with an extremely small heavy metals contamination as a result of the manufacture based on lyocell spinning solution 104. The medical product may be a medication delivery system, a disinfection system, a cleaning system or a separation system (for instance separating a patient with regard to an environment). The high intra-fibrillar fluid accommodation capability of the fabric 102 may remove fluid from the patient, and may at the same time provide an active agent 272, 276 to the patient.

A fabric 102 according to an exemplary embodiment of the invention may be used for dryer sheets. Dryer sheets may be added to a dryer for drying clothes so as to release one or more active agents (such as a fragrance, a disinfectant, a drying promoter, etc.) during the drying procedure. For such an application, the effect may be used that in a non-swollen state of the fiber fabric 102 the mechanical tension conditions in an interior of the fabric 102 are different than in the swollen state of the fabric 102. By varying fiber diameters of the fibers 108 of the fabric 102 it may be ensured that certain cavities 274 with embedded particles 272, 276 are less under mechanical tension than when the sheet as a whole is swollen. This may trigger the release of an active agent 272, 276. Moreover, a pressure generated by swelling or shrinking may press a viscous liquid droplet out of the fabric 102. Descriptively speaking, the swelling or shrinking process may be considered as a mechanical squeezing of the fabric 102 promoting release of an active agent 272, 276.

A fabric 102 according to an exemplary embodiment of the invention may be used for a face mask with additional active agent 272, 276. A face mask manufactured based on fabric 102 can be enriched with one or more active agent 272, 276 during its production. Also multiple active agents 272, 276 to be released in different (in particular non-overlapping) time intervals may be integrated in one and the same face mask. For instance, release of an active agent 272, 276 may be triggered by a continuous drying procedure during use of the face mask. In other words, when the face mask is used, moisture stored therein at the beginning may evaporate, which may result in a shrinkage of the fibers 108 of the fabric 102. With a corresponding fabric design, release of an active agent 272, 276 may be initiated when a certain shrinkage level has been reached. In this context, the pronounced moisture spreading capability of the fabric 102 may be used for homogeneously distributing moisture and homogeneously drying the face mask during use. This is in particular advantageous in view of the fact that certain portions of the human face skin tend to absorb more moisture than other skin portions. The fabric 102 may balance out such inhomogeneities and may ensure a homogeneous moisture distribution over the entire face mask as a result of the high fluid spreading speed of the fabric 102. Moreover, a shrinking speed of the fibers 108 (which may be adjusted by a control of the process parameters of the manufacturing method) may adapt the active agent release speed of the fabric 102 to the active agent absorption speed of the face skin. Multilevel release of different active agents 272, 276 one after the other may be adjusted as well. For instance, at first, a first active agent 272 located at a surface of the fabric 102 may be supplied to the skin. Only thereafter, continued shrinkage of the fibers 108 may trigger release of a second active agent 276 which had been locked within cavities 274 prior to shrinkage. Homogeneity of the drying procedure may be promoted by forming a multilayer fabric 102 and/or by controlling merging homogeneity. When the degree of homogeneity of the fiber structure is high, a proper dynamics of the moisture equilibration during the drying procedure can be ensured.

Summarizing, in particular one or more of the following adjustments may be made according to exemplary embodiments of the invention:
- a low homogeneous fiber diameter may allow to obtain a high smoothness of the fabric 102
- multilayer fabric 102 with low average fiber diameters may allow to obtain a high fabric thickness at a low fabric density
- equal absorption curves of the functionalized layers can allow to obtain a homogeneous humidity and fluid accommodation behavior, as well as a homogenous behavior in terms of fluid release
- the described connection of layers 200, 202 of fabric 102 allows to design products with low linting upon layer separation
- it is also possible to differently functionalize single layers 200, 202 so that products with anisotropic properties are obtained (for instance for wicking, oil accommodation, water accommodation, cleanability, roughness).

Finally, it should be noted that the above-mentioned embodiments illustrate rather than limit the invention, and that those skilled in the art will be capable of designing many alternative embodiments without departing from the scope of the invention as defined by the appended claims. In the claims, any reference signs placed in parentheses shall not be construed as limiting the claims. The words "comprising" and "comprises", and the like, do not exclude the presence of elements or steps other than those listed in any claim or the specification as a whole. The singular reference of an element does not exclude the plural reference of such elements and vice-versa. In a device claim enumerating several means, several of these means may be embodied by one and the same item of software or hardware. The mere fact that certain measures are recited in mutually different dependent claims does not indicate that a combination of these measures cannot be used to advantage.

## Claims

1. A nonwoven cellulose fiber fabric (102), in particular directly manufactured from lyocell spinning solution (104), wherein the fabric (102) comprises a network of substantially endless fibers (108), and wherein the fabric (102) further comprises:
a plurality of first pores (260) delimited between a first plurality of the fibers (108) and having sizes (280) within a first size range;
a plurality of second pores (264) delimited between a second plurality of the fibers (108) and having sizes (282) within a second size range;
wherein the first size range encompasses sizes (280) being smaller than sizes (282) encompassed by the second size range.

2. The fabric (102) according to claim 1, comprising at least one of the following features:
wherein at least part of the fibers (108) form part of both the first plurality of the fibers (108) and the second plurality of the fibers (108);
wherein at least part of the fibers (108) form part of only the first plurality of the fibers (108) or only the second plurality of the fibers (108);
wherein the plurality of first pores (260) is suitable for retaining and/or releasing first particles (262);
wherein the plurality of second pores (264) is suitable for retaining and/or releasing second particles (266);
wherein the first size range and the second size range have no size (280, 282) in common;
wherein the fibers (108) are configured so that the respective size range of at least one of the plurality of first pores (260) and the plurality of second pores (264) is modified by at least one of the group consisting of swelling and shrinking of the fibers (108) depending on a humidity state of the fibers (108).

3. The fabric (102) according to claim 1 or 2, configured so the first particles (262) are enabled to selectively enter into or leave the first pores (260).

4. The fabric (102) according to claim 3, comprising at least one of the following features:
wherein the first size range of the first pores (260) is configured so that first particles (262) with a diameter in a range between 0.5 µm and 500 µm, in particular in a range between 3 µm and 300 µm, are enabled to enter into or leave the first pores (260) in a dry state of the fibers (108);
wherein the first size range of the first pores (260) is configured so that first particles (262) with a diameter in a range between 0.5 µm and 500 µm, in particular in a range between 3 µm and 300 µm, are disabled to enter into or leave the first pores (260) in a wet state of the fibers (108).

5. The fabric (102) according to any of claims 1 to 4, comprising at least one of the following features:
the plurality of first pores (260) are provided over the entire fabric (102) and the plurality of second pores (264) are provided only over a subsection of the fabric (102);
comprising a medium (262, 266, 272, 276), in particular comprising at least 1 mass percent of a medium (262, 266, 272, 276) related to a mass of the entire fabric (102), filling at least one of the plurality of first pores (260) and the plurality of second pores (264);
comprising an active agent (272) accommodated in the plurality of second pores (264);
wherein the endless fibers (108) have an amount of fiber ends per volume of less than 10,000 ends/cm³, in particular less than 5,000 ends/cm³;
wherein the fibers (108) differ concerning fiber diameter so that a ratio between an average diameter of the 10% thinnest fibers (108) and an average diameter of the 10% thickest fibers (108) is more than 0.01, in particular is more than 0.05, more particularly is more than 0.1;
wherein at least 80 mass percent of the fibers (108) have an average fiber diameter in a range between 1 µm and 40 µm, in particular between 3 µm and 15 µm;
wherein the fibers (108) have a copper content of less than 5 ppm and/or have a nickel content of less than 2 ppm;
wherein the fabric (102) is configured so that a wicking speed is at least 0.25 g water/g fabric/s;
wherein the fabric (102) is configured so that cavities (274) defined between at least part of the pores (260, 264) undergo a diameter reduction of at least 20%, in particular at least 30%, from a substantially dry, conditioned state with between 5% and 15% moisture content to a wet state of the fabric with at least more than 20% moisture content (102);
wherein at least part of the fibers (108) are integrally merged at merging positions (204);
wherein the fabric (102) comprises a first fabric portion (268) having the plurality of first pores (260), and comprises a second fabric portion (270) being different from the first fabric portion (268) and having the plurality of second pores (264).

6. A method of manufacturing nonwoven cellulose fiber fabric (102) directly from lyocell spinning solution (104), wherein the method comprises
extruding the lyocell spinning solution (104) through at least one jet (122) with orifices (126) supported by a gas flow (146) into a coagulation fluid (106) atmosphere to thereby form substantially endless fibers (108);
collecting the fibers (108) on a fiber support unit (132) to thereby form the fabric (102);
adjusting process parameters so that the fabric (102) is formed with:
a plurality of first pores (260) delimited between a first plurality of the fibers (108) and having sizes (280) within a first size range;
a plurality of second pores (264) delimited between a second plurality of the fibers (108) and having sizes (282) within a second size range;
wherein the first size range encompasses sizes (280) being smaller than sizes (282) encompassed by the second size range.

7. The method according to claim 6, wherein the method further comprises at least one of the group consisting of:
adding moisture to at least a portion of the fabric (102) to thereby decrease at least one of the size ranges by moisture-based swelling of at least part of the fibers (108);
removing moisture from at least a portion of the fabric (102) to thereby increase at least one of the size ranges by moisture-based shrinkage of at least part of the fibers (108);
adjusting a humidity state of the fibers (108) for modifying the respective size range of at least one of the group consisting of the plurality of first pores (260) and the plurality of second pores (264);
forming the plurality of second pores (264) after collecting the fibers (108) on the fiber support unit (132), in particular by hydroentanglement.

8. The method according to claim 6 or 7, comprising at least one of the following features:
wherein each of the plurality of first pores (260) and/or cavities (274) in fluid communication with at least part of the first pores (260) is delimited between at least three fibers (108) of the fabric (102);
wherein each of the plurality of second pores (264) and/or cavities (274) in fluid communication with at least part of the second pores (264) is formed by hydro-entanglement.

9. The method according to any of claims 6 to 8, wherein the method further comprises further processing the fibers (108) and/or the fabric (102) *in situ* after collection on the fiber support unit (132), in particular by at least one of the group consisting of hydro-entanglement, needle punching, impregnation, steam treatment with a pressurized steam, steam treatment with a pressurized steam, and calendering.

10. A device (100) for manufacturing nonwoven cellulose fiber fabric (102) directly from lyocell spinning solution (104), wherein the device (100) comprises:
at least one jet (122) with orifices (126) configured for extruding the lyocell spinning solution (104) supported by a gas flow (146);
a coagulation unit (128) configured for providing a coagulation fluid (106) atmosphere for the extruded lyocell spinning solution (104) to thereby form substantially endless fibers (108);
a fiber support unit (132) configured for collecting the fibers (108) to thereby form the fabric (102);
a control unit (140) configured for adjusting process parameters so that the fabric (102) is formed with:
a plurality of first pores (260) delimited between a first plurality of the fibers (108) and having sizes (280) within a first size range;
a plurality of second pores (264) delimited between a second plurality of the fibers (108) and having sizes (282) within a second size range;
wherein the first size range encompasses sizes (280) being smaller than sizes (282) encompassed by the second size range.

11. The device (100) according to claim 10, comprising a further processing unit (134) for forming the plurality of second pores (264) after collecting the fibers (108) on the fiber support unit (132), in particular by hydroentanglement or needlepunching.

12. A method of controlling release of an active agent (272, 276), comprising:
providing a nonwoven cellulose fiber fabric (102) comprising a network of substantially endless fibers (108), a plurality of pores (260, 264) delimited between the fibers (108), and the active agent (272) retained in at least part of the pores (260, 264) and/or in connected cavities (274) in the fabric (102);
adjusting a condition of the fabric (102) for triggering release of the active agent (272) out of the pores (260, 264) and/or the cavities (274).

13. The method according to claim 12, wherein the method further comprises at least one of the group consisting of:
adjusting the condition comprises adjusting a humidity state of the fibers (108) for triggering one of the group consisting of a swelling and a shrinkage of the fibers (108) to thereby control release of the active agent (272) out of the pores (260, 264) and/or the cavities (274);
providing a further active agent (276) in at least part of the pores (260, 264) and/or the cavities (274), and adjusting a condition of the fabric (102), in particular a humidity state of the fibers (108), for triggering release of the further active agent (276) out of the pores (260, 264) and/or the cavities (274) after having completed the release of the active agent (272) out of the pores (260, 264) and/or the cavities (274).

14. A method of using a nonwoven cellulose fiber fabric (102) according to any of claims 1 to 5 for at least one of the group consisting of a wipe, a dryer sheet, a filter, a hygiene product, a medical application product, a geotextile, agrotextile, clothing, a product for building technology, an automotive product, a furnishing, an industrial product, a product related to beauty, leisure, sports or travel, and a product related to school or office.

15. A product or composite, comprising a fabric (102) according to any of claims 1 to 5.
